# EUROPEAN PATENT SPECIFICATION

(11) **EP 4 484 570 B1**
(45) Date of publication and mention of the grant of the patent: **23.09.2026**
(21) Application number: 23760098.6
(22) Date of filing: 24.02.2023
(51) Int. Cl.: C12Q 1/6825, C12M 1/00, C12M 1/34, C12N 15/11, C12Q 1/6834, G01N 21/17, G01N 21/49, G01N 21/59, G01N 21/64, G01N 33/542, G01N 33/543, C12Q 1/6816

(54) **DETECTION METHOD FOR ANALYTE AND DETECTION SYSTEM FOR ANALYTE**
DETEKTIONSVERFAHREN FÜR EINEN ANALYTEN UND DETEKTIONSSYSTEM FÜR EINEN ANALYTEN
PROCÉDÉ DE DÉTECTION DE SUBSTANCE À ANALYSER ET SYSTÈME DE DÉTECTION POUR SUBSTANCE À ANALYSER

(30) Priority: 24.02.2022 JP 2022026950
(43) Date of publication of application: 01.01.2025
(73) Proprietor: University Public Corporation Osaka, Osaka City 545-0051 (JP)
(72) Inventor: IIDA, Takuya, Sakai-shi, Osaka 599-8531 (JP); TOKONAMI, Shiho, Sakai-shi, Osaka 599-8531 (JP); TAKAGI, Yumiko, Sakai-shi, Osaka 599-8531 (JP); HAYASHI, Kota, Sakai-shi, Osaka 599-8531 (JP); TOYOUCHI, Shuichi, Sakai-shi, Osaka 599-8531 (JP)
(74) Representative: Bals & Vogel Patentanwälte PartGmbB
(86) International application number: PCT/JP2023/006708
(87) International publication number: WO 2023/163099

(56) References cited:
- WO-A1-2014/192937
- WO-A1-2015/170758
- WO-A1-2015/170758
- WO-A1-2021/040021
- WO-A1-2021/040021
- US-A1- 2010 330 578

## Description

### TECHNICAL FIELD

The present disclosure relates to a method for detecting an analyte and a system for detecting an analyte, and relates specifically to a technique for detecting an analyte that may be contained in a sample by light irradiation.

### BACKGROUND ART

WO 2014/192937 (PTL 1) and WO 2021/040021 (PTL 2) disclose techniques for detecting an analyte that may be contained in a sample by light irradiation. WO 2017/213107 (PTL 3) discloses a technique for accumulating nanocapsules containing metal nanoparticles.

### CITATION LIST

### PATENT LITERATURE

PTL 1: WO 2014/192937
PTL 2: WO 2021/040021
PTL 3: WO 2017/213107

### SUMMARY OF INVENTION

### TECHNICAL PROBLEM

A technique for detecting a very small amount of an analyte and shortening time for detecting an analyte, in other words, a technique that enables detecting an analyte with high sensitivity rapidly, is always requested.

The present disclosure has been completed to achieve the above-mentioned object. One of the objects of the present disclosure is to detect an analyte with high sensitivity rapidly.

### SOLUTION TO PROBLEM

(1) A method for detecting an analyte according to a first aspect of the present disclosure comprises the first to third steps. The first step is preparing a liquid sample comprising nanoparticles each modified with first host molecules to be specifically bound to the analyte and microparticles each modified with second host molecules to be specifically bound to the analyte. The second step is irradiating the liquid sample with non-resonant light outside a wavelength region of electronic resonance of the nanoparticles and outside a wavelength region of electronic resonance of the microparticles. The third step is detecting an analyte based on an output signal from a light receiver receiving light from the liquid sample. The preparing (the first step) includes: combining the nanoparticles and the microparticles that are different from each other in size and material so that (i) the nanoparticles each have such a size as to be diffused in the liquid sample by Brownian motion, (ii) the microparticles each have such a size as to receive dissipation force due to the Mie scattering of the non-resonant light, and (iii) the nanoparticles each include material that generates thermal convection in the liquid sample by the irradiating the non-resonant light.
(2) The preparing (the first step) further comprises combining the nanoparticles and the microparticles different from each other in concentration so that the average interparticle distance of the nanoparticles is shorter than the average interparticle distance of the microparticles.
(3) The preparing further includes mixing the nanoparticles and the microparticles uniformly.
(4) The method for detecting the analyte further comprises: retaining the liquid sample on a substrate; and adjusting, after the retaining, the irradiation position of the non-resonant light so that the focal point of the non-resonant light is positioned behind the substrate in the non-resonant light propagation direction.
(5) The method for detecting the analyte further comprises: adjusting the irradiation position of the non-resonant light so that the focal point of the non-resonant light is located behind a substrate in the non-resonant light propagation direction; and retaining, after the adjusting, the liquid sample on the substrate.
(6) The method for detecting the analyte further comprises retaining the liquid sample in a well provided on the substrate. The irradiating (the second step) includes condensing the non-resonant light with a condensing lens including a correction collar. The method for detecting the analyte further comprises adjusting the correction collar so as to correct the spherical aberration in accordance with the thickness of the substrate and the depth of the well.
(7) The analyte is labeled with fluorescent dye. The detecting (the third step) includes: measuring the fluorescence intensity of the liquid sample; and detecting the analyte based on a variation in the fluorescence intensity accompanying the irradiating the non-resonant light.
(8) The analyte is a DNA including multiple bases. The detecting comprises detecting a single nucleotide polymorphism of the DNA.
(9) The detecting includes: measuring the absorbance spectrum of the liquid sample; and detecting the analyte based on a variation in the absorbance spectrum accompanying the irradiating the non-resonant light.
(10) The detecting includes: photographing the accumulation region of the accumulated nanoparticles and microparticles; and detecting the analyte based on the area of the accumulation region.
(11) The liquid sample is a highly viscous liquid sample including impurities. The detecting includes specifically detecting the analyte from the highly viscous liquid sample.
(12) A system that detects an analyte according to a second aspect of the present disclosure comprises: nanoparticles each modified with first host molecules to be specifically bound to the analyte; microparticles each modified with second host molecules to be specifically bound to the analyte; a light source that emits non-resonant light outside a wavelength region of the electronic resonance of the nanoparticles and outside a wavelength region of the electronic resonance of the microparticles; a light receiver that receives light from the liquid sample irradiated with the non-resonant light and including the nanoparticles and the microparticles; and a processor that executes detection processing that detects the analyte based on an output signal from the light receiver. The nanoparticles and the microparticles that are different from each other in size and material are combined so that (i) the nanoparticles each have such a size as to collide with dispersion medium molecules in the liquid sample to cause Brownian motion, (ii) the microparticles each have such a size as to receive dissipative force due to the Mie scattering of the non-resonant light, and (iii) the nanoparticles each include material that generates thermal convection in the liquid sample by irradiating the non-resonant light.

### ADVANTAGEOUS EFFECTS OF INVENTION

The present disclosure enables detecting an analyte with high sensitivity rapidly.

### BRIEF DESCRIPTION OF DRAWINGS

Fig. 1 illustrates a first probe particle in the present embodiment.
Fig. 2 illustrates a second probe particle in the present embodiment.
Fig. 3 illustrates for describing the detection principle of target DNA with the first probe particles and the second probe particles.
Fig. 4 shows an example of the entire configuration of a system for detecting an analyte according to the present embodiment.
Fig. 5 shows another example of the entire configuration of a system for detecting an analyte according to the present embodiment in the upright configuration.
Fig. 6 is a perspective view showing the configuration of a detection kit schematically.
Fig. 7 shows an image of an actually manufactured detection kit.
Fig. 8 shows diagrams for describing aspects in which the detection kit is irradiated with laser light.
Fig. 9 is a flow chart showing a procedure for subjecting the analyte to detection processing.
Fig. 10 is an illustration for describing dissipative force acting on the probe particles.
Fig. 11 shows the results of the simulation of the Brownian motion of the probe particles.
Fig. 12 is a conceptual diagram for describing the average interparticle distances of the probe particles.
Fig. 13 illustrates the evaluation results of the combinations of probe particles.
Fig. 14 shows an example of images photographed with a camera in Example 2.
Fig. 15 shows an example of absorbance spectra measured with a spectroscope in Example 2.
Fig. 16 shows the dependence of the absorbance differences on the target concentrations.
Fig. 17 shows the dependence of the accumulation areas of the probe particles on the target concentrations.
Fig. 18 is a table summarizing the measurement conditions in Example 3.
Fig. 19 shows transmission images and fluorescence images of a sample 1.
Fig. 20 shows transmission images and fluorescence images of a sample 2.
Fig. 21 shows transmission images and fluorescence images of a sample 3.
Fig. 22 shows transmission images and fluorescence images of a sample 4.
Fig. 23 shows transmission images and fluorescence images of a sample 5.
Fig. 24 shows transmission images and fluorescence images of a sample 6.
Fig. 25 shows transmission images and fluorescence images of a sample 7.
Fig. 26 shows an example of the measurement results of the dependent of the fluorescence intensities on the positions.
Fig. 27 shows an example of calibration curves acquired from the fluorescence intensities shown in Fig. 26.
Fig. 28 shows images in which it is photographed how fluorescence is enhanced on the surfaces of metal nanoparticles contained in the probe particles.
Fig. 29 is a first set of images showing the dependence of the fluorescence intensities on the base sequences.
Fig. 30 is a second set of images showing the dependence of the fluorescence intensities on the base sequences.
Fig. 31 shows transmission images and fluorescence images of low-concentration samples.
Fig. 32 is a graph summarizing the measurement results of the fluorescence intensities at concentrations of target DNA 9.
Fig. 33 is an illustration for describing a third improvement.
Fig. 34 is an illustration for describing eight target DNAs.
Fig. 35 is a graph summarizing the measurement results of the fluorescence intensities of the eight target DNAs.
Fig. 36 shows the detection results of target DNAs in highly viscous samples.

### DESCRIPTION OF EMBODIMENTS

With reference to drawings, the embodiments of the present disclosure will be described in detail hereinafter. The same or equivalent components are indicated with the same reference characters in the figures, without redundant description.

### <Description of terms>

The phrase "nanometer order" as used in the present disclosure and the embodiments thereof includes the range of 1 nm to 1000 nm (1 µm). The phrase "micrometer order" includes the range of 1 µm to 1000 µm (1 mm). Accordingly, the phrase "from the nanometer order to the micrometer order" includes the range of 1 nm to 1000 µm. The phrase "from the nanometer order to the micrometer order" typically means the range of several nanometers to several hundred micrometers, and can mean preferably the range of 100 nm to 100 µm and more preferably 1 µm to several dozen micrometers.

The "sample" as used in the present disclosure and the embodiments thereof means a substance that contains or may contain an analyte. The sample can be a biological sample, for example, from an animal (a human, a bovine, a horse, a pig, a goat, a chicken, a rat, or a mouse). The biological sample can include, for example, blood, tissue, cells, a secretion, and a body fluid. The "sample" may include a dilution thereof or an isolate therefrom (for example, serum or blood plasma). The "liquid sample" is liquid containing the sample.

The "analyte" as used in the present disclosure and the embodiments thereof means a substance as an object to be detected having a size from the nanometer order to the micrometer order. The analyte may have any shape, for example, a spherical, oval-spherical, or rod-like shape. If the analyte has an oval-spherical shape, at least one of the lengths in the minor axis and major axis directions of the oval sphere only has to be in the range from the nanometer order to the micrometer order. If the analyte has a rod-like shape, at least one of the width and the length of the rod only has to be in the range from the nanometer order to the micrometer order.

Examples of the analyte include cells, microorganisms (bacteria and fungi), vesicles (exosomes, microvesicles, and apoptotic vesicles), biopolymers (proteins, nucleic acids, lipids, and polysaccharides), antigens (allergens), and viruses. Specific examples of the protein include antibodies such as CD9, CD63, CD80, and CD81, subjected to CD (cluster of differentiation) classification; cytokines such as IL-6; and albumin. Examples of the nucleic acids include DNAs and RNAs. Specific examples of the nucleic acids can include cell-free DNAs (cfDNAs), circulating tumor DNAs (ctDNAs), derived from cancer cells, messenger RNAs (mRNAs), and microRNAs (miRNAs). The analyte is not, however, limited to substances derived from living bodies (biological substances), and may be resin beads, metal nanoparticles, metal nanoparticle assemblies, metal nanoparticle condensed structure bodies, semiconductor nanoparticles, and organic nanoparticles.

The "fine particles" as used in the present disclosure and the embodiments thereof means a substance having a size from the nanometer order to the micrometer order. The fine particles include nanoparticles and microparticles.

The "nanoparticles" as used in the present disclosure and the embodiments thereof means particles having a size in the nanometer order. The nanoparticles may be metal nanoparticles, semiconductor nanoparticles, or organic nanoparticles.

The "metal nanoparticles" as used in the present disclosure and the embodiments thereof means metal particles having a size in the nanometer order. The metal nanoparticles can include metal nanoparticle assembly and metal nanoparticle condensed structure body. The "metal nanoparticle assembly" is an assembly formed by aggregating metal nanoparticles. The "metal nanoparticle condensed structure body" is, for example, a structure in which metal nanoparticles are immobilized on the surface of beads through interactive sites, the metal nanoparticles are spaced and disposed at intervals equal to or shorter than the diameter of the metal nanoparticles.

The shape of the metal nanoparticles is not limited to a spherical shape, and may be, for example, an oval-spherical shape or a rod-like shape. If the metal nanoparticles have an oval-spherical shape, at least one of the lengths in the major axis and minor axis directions of the oval sphere only has to be in the nanometer order. If the metal nanoparticles has a rod-like shape, at least one of the width and the length of the rod only has to be in the nanometer order.

The "semiconductor nanoparticles" as used in the present disclosure and the embodiments thereof means semiconductor particles having a size in the nanometer order. The "organic nanoparticles" as used in the present disclosure and the embodiments thereof means particles having a size in the nanometer order and comprising an organic compound. The semiconductor nanoparticles and the organic nanoparticles may also have any shape like the metal nanoparticles, and may have, for example, a spherical, oval-spherical, or rod-like shape.

The "microparticles" as used in the present disclosure and the embodiments thereof means particles having a size in the micrometer order. The microparticles may be resin beads, magnet beads, or PM (particulate matter). The "resin beads" are particles with a size in the micrometer order comprising resin. The "magnet beads" are magnetic particles with a size in the micrometer order (polymer particles in which a magnetic substance is dispersed or embedded). The "PM" is a particulate substance having a size in the micrometer order. The material of the microparticles is not limited to resin and an organic compound, but may be, for example, metal or a semiconductor.

The "host molecules" as used in the present disclosure and the embodiments thereof mean a substance that can be specifically bound (or may be specifically attached) to the analyte. Examples of the combination of the host molecules and the analyte include an antigen and an antibody; a sugar chain and a protein; a lipid and a protein; a low-molecular weight compound (ligand) and a protein; a protein and another protein; and a single-stranded DNA and another single-stranded DNA. If any one substance is the analyte among both these substances having specific affinity for each other, the other can be used as the host molecules.

For example, if the analyte is a single-stranded DNA (target DNA), the host molecules are the other single-stranded DNA (probe DNA). An anti-DNA antibody to be specifically bound to DNA (for example, an anti-dsDNA to be specifically bound to a double-stranded DNA or an anti-ssDNA to be specifically bound to a single-stranded DNA) can also be used as the host molecules.

If an antigen is the analyte, an antibody can be used as the host molecules. On the contrary, if an antibody is the analyte, an antigen can be used as the host molecules. The antigen can include allergens, microorganisms (bacteria and fungi), viruses, and vesicles. The change of the type of the antibody also enables changing the type of a detectable allergen, microorganism, or virus. Accordingly, the allergen, the microorganism, or the virus detectable by the present disclosure may be of any type. If the analyte is a heavy metal, a substance that enables collecting heavy metal ions can be used as the host molecules.

The host molecules are immobilized on the surfaces of the fine particles by the interaction between the host molecules and the fine particles. The type of the interaction for immobilizing the host molecules on the surfaces of the fine particles depends on the type of the fine particles. The interaction includes covalent bonding, ionic bonding, metallic bonding, van der Waals force, electrostatic interaction, hydrophobic interaction, intermolecular force (for example, hydrogen bonding), and adsorption force.

The term "light-induced force" as used in the present disclosure and the embodiments thereof is used as a generic term for dissipative force, gradient force, and inter-object light-induced force. Dissipative force is force generated by giving the momentum of light to a substance in a dissipative process such as light scattering or light absorption. Gradient force is force that moves a substance to an electromagnetic potential stability point if the substance subjected to light-induced polarization is placed in a nonuniform electromagnetic field. Inter-object light-induced force is the sum of force caused by a vertical electric field generated by induced polarization in multiple photoexcited substances and force caused by a lateral electric field (radiation field). Light-induced polarization is electrical polarization caused by exciting electrons in a substance with light.

The "visible light" as used in the present disclosure and the embodiments thereof means light in a wavelength region of 400 to 700 nm. The "infrared light" means light in a wavelength region of 700 nm to 1,000 µm (1 mm), preferably 700 nm to 2,500 nm, and more preferably 700 to 1,400 nm. The "white light" means light in a wavelength region from the ultraviolet region to the near-infrared region (for example, a wavelength region of 200 to 1100 nm).

"Resonant light" as used in the present disclosure and the embodiments thereof means light that causes high light-induced polarization in the fine particles by incidence of the light on the fine particles. The resonant light has a wavelength in a wavelength region of electronic resonance of the fine particles. Meanwhile, the "non-resonant light" means light that causes low light-induced polarization in the fine particles by incidence of the light on the fine particles. The non-resonant light has a wavelength outside a wavelength region of electronic resonance of the fine particles.

If the fine particles are fine metal particles, the "wavelength region of electronic resonance of the fine particles" is a wavelength region corresponding to the full width at half maximum of the peak of the localized surface plasmon resonance. The wavelength region in which the localized surface plasmon resonance is caused on the fine metal particles depends on the size of the fine metal particles. If the fine metal particle are metal nanoparticles, the wavelength region is typically the wavelength region of visible light at 400 to 700 nm. Light "outside the wavelength region of electronic resonance of the fine particles" (non-resonant light) is typically infrared light at a wavelength longer than 700 nm.

If the fine particles are fine semiconductor particles or fine organic particles, the "wavelength region of electronic resonance of the fine particles" is a wavelength region in which interband transition or exciton resonance (resonance of electron-positive hole pairs) occurs. If the fine particles are fine organic particles (for example, microparticles comprising organic material such as polystyrene), the wavelength region is a wavelength region at a wavelength shorter than 400 nm. Light "outside wavelength region of electronic resonance of the fine particles" (non-resonant light) is typically visible light at a wavelength longer than 400 nm.

### [Embodiments]

With reference to the drawings, the embodiments of the present disclosure will be described in detail hereinafter. The same or equivalent components are indicated with the same reference characters in the figures, without redundant description. In the following description, the x direction and the y direction indicate horizontal directions. The x direction is orthogonal to the y direction. The z direction indicates the vertical direction. The gravity is downward in the z direction. The phrase "upward in the z direction" is abbreviated as "upward", and the phrase "downward in the z direction" is abbreviated as "downward".

### <Detection principle of analyte>

An example in which the analyte is DNA will be described. The DNA is described as "target DNA". The target DNA is a single-stranded DNA having a predetermined base sequence. In the present embodiment, two types of probe particles are used for detecting the target DNA.

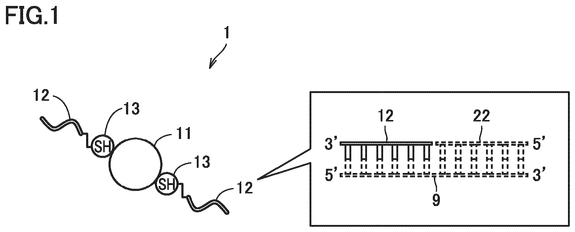

Fig. 1 illustrates a probe particle 1 in the present embodiment. The probe particle 1 includes, for example, a metal nanoparticle 11, host molecules 12, and interactive sites 13.

Metal nanoparticles 11 are, for example, gold nanoparticles having a diameter of 30 nm. As described above, the wavelength at which light causes localized surface plasmon resonance on gold nanoparticles in liquid (for example, in water) is generally in the wavelength region of visible light (typically 400 to 700 nm). The use of light out of the wavelength region in which light causes localized surface plasmon resonance (for example, infrared light) may however be desirable in some application fields. In the present embodiment, metal nanoparticles 11 are irradiated with such non-resonant light. Also in this case, localized surface plasmon resonance is induced on the surfaces of the gold nanoparticles. As long as localized surface plasmon resonance can be caused on the metal nanoparticles 11, metal nanoparticles 11 may be metal nanoparticles other than gold nanoparticles, and may be, for example, silver nanoparticles.

As long as metal nanoparticles 11 have a size (diameter) in the nanometer order, metal nanoparticles 11 have any size, but have preferably a size of several dozen nanometers or more like this example. It is because metal nanoparticles that can be captured by light-induced force generated by irradiating non-resonant light at a typical light intensity (around several hundred milliwatt) are supposed to have a size of several dozen nanometers or more.

Metal nanoparticles 11 are an example of "particles" according to the present disclosure. The "particles" according to the present disclosure may be semiconductor nanoparticles or organic nanoparticles. The "nanoparticles" according to the present disclosure may contain two or three types of metal nanoparticles, semiconductor nanoparticles, and organic nanoparticles.

Host molecules 12 are a substance to be specifically bound to target DNA 9. In the embodiment, host molecules 12 are a probe DNA having a base sequence complementary to a base sequence on the 5'-end side of target DNA 9 between the 3'-end and the 5'-end. The host molecules 12 correspond to "first host molecules" according to the present disclosure.

Interactive sites 13 are moieties through which metal nanoparticle 11 can interact with host molecules 12. Interactive sites 13 immobilize host molecules 12 on the surface of metal nanoparticle 11. The surface of metal nanoparticle 11 is modified with host molecules 12 thereby. If metal nanoparticles 11 are gold nanoparticles, and host molecules 12 are a probe DNA, interactive sites 13 are, for example, thiol group on the 3'-terminus side of the probe DNA (represented by SH).

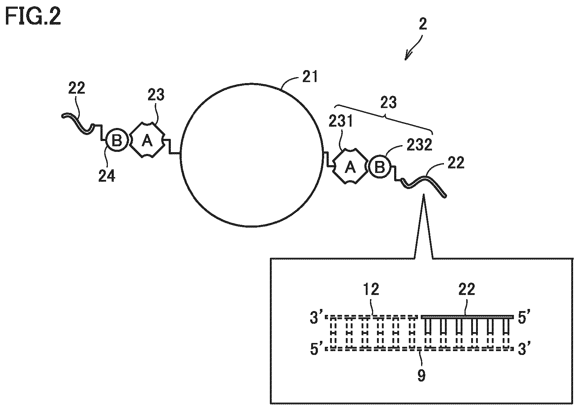

Fig. 2 illustrates a probe particle 2 in the present embodiment. Probe particle 2 includes a microparticle 21, host molecules 22, and interactive sites 23.

Microparticles 21 are, for example, resin beads with a diameter of 2 µm comprising polystyrene. The material of microparticles 21 may be another resin such as acrylic resins, polyolefins, polyethylene, or polypropylene. As long as microparticles 21 have a size (diameter) in the micrometer order, microparticles 21 may have any size, but may have a size equivalent to that of common resin beads (so-called latex beads) (around 1 to 5 µm).

Host molecules 22 are a substance to be specifically bound to the target DNA 9. In the present embodiment, host molecules 22 are a probe DNA having a base sequence complementary to a base sequence on the 3'-end side of the target DNA 9 between the 3'-end and the 5'-end. Host molecules 22 correspond to "second host molecules" according to the present disclosure.

Interactive sites 23 are moieties through which microparticles 21 can interact with host molecules 22. Interactive sites 23 immobilize host molecules 22 on the surfaces of microparticles 21. The surfaces of microparticles 21 are modified with host molecules 22 thereby. If microparticles 21 are resin beads, and host molecules 22 are a probe DNA, interactive sites 23 include, for example, avidin 231 (represented by A) and biotin 232 (represented by B). Avidin 231 is immobilized on the surfaces of microparticles 21 by the interaction between avidin 231 and microparticles 21. Biotin 232 is bound to host molecules 22 (5'-end of the probe DNA) (biotinylation). The surfaces of microparticles 21 are modified with host molecules 22 by a strong affinity between avidin 231 and biotin 232.

Fig. 1 shows an example in which the surface of metal nanoparticle 11 is modified with two host molecules 12, to prevent the figure from being complicated. The surface of metal nanoparticle 11 is however actually modified with more host molecules 12. The same is applied to host molecules 22 with which the surface of microparticle 21 is modified.

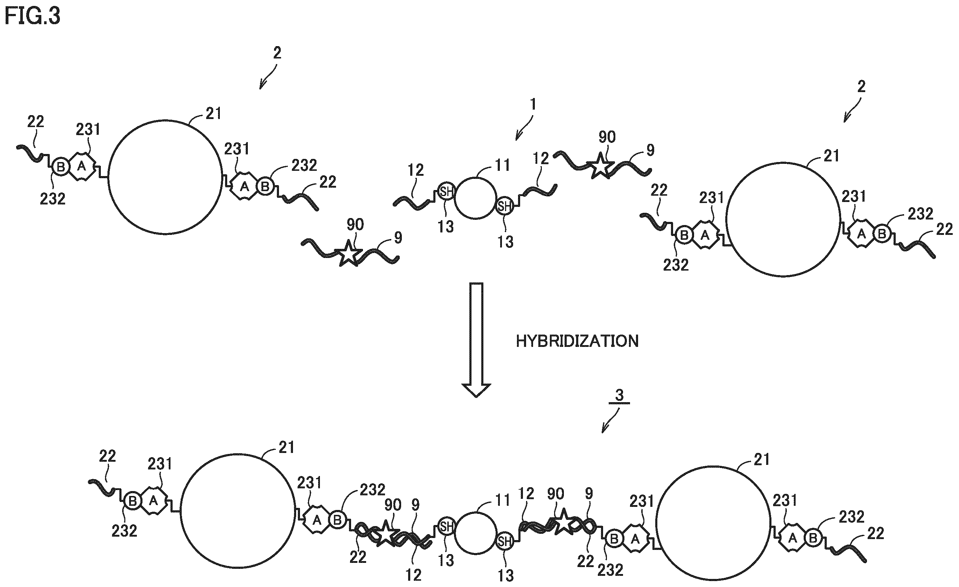

Fig. 3 is an illustration for describing the detection principle of target DNA 9 with the probe particles 1 and 2. In this example, target DNA 9 is labeled with fluorescent dye 90 (in detail, Alexa Flour(R) 488, which is available from Thermo Fisher Scientific K.K.).

The introduction of probe particles 1 and probe particles 2 into a sample containing target DNA 9 hybridizes host molecules 12 of probe particles 1 with target DNA 9, and hybridizes host molecules 22 of probe particles 2 with target DNA 9. This aggregates probe particles 1 and probe particles 2 to form an aggregate 3. Aggregate 3 grows to approximately a certain size to be optically detectable (photographable with a camera 57, for example, in an example described below). Accordingly, the optical detection of aggregate 3 shows that the sample contains target DNA 9.

The "hybridization" means reassociation reaction between two single-stranded DNAs. In the present embodiment, two single-stranded DNAs having complementary base sequences form a double-stranded DNA. The hybridization is not, however, limited thereto, and includes the formation of a double strand from one single-stranded DNA and RNA or from two RNAs.

### <Entire configuration of system>

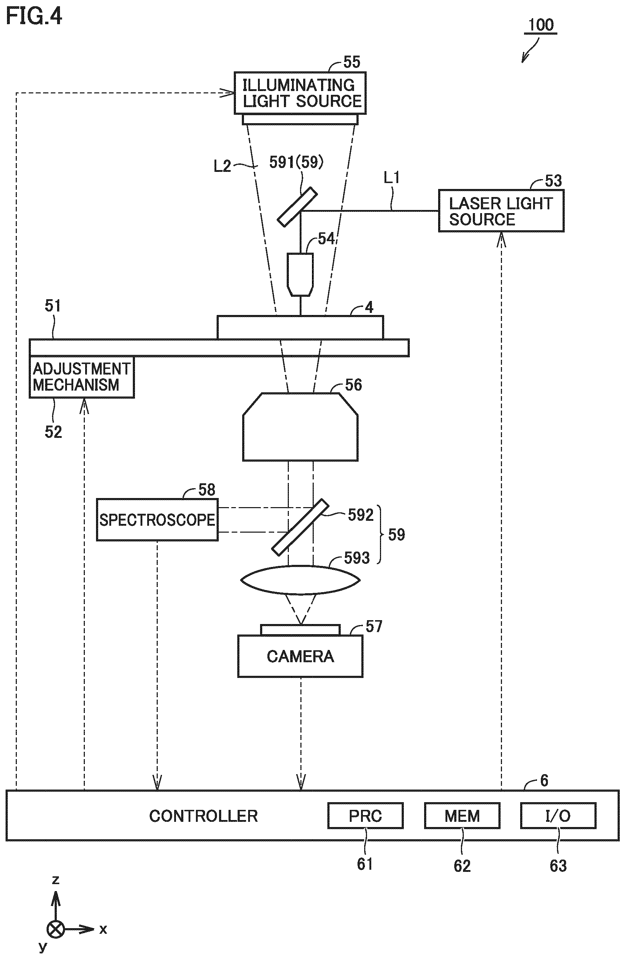

Fig. 4 shows an example of the entire configuration of a system for detecting target DNA 9 according to the present embodiment. A detection system 100 comprises a detection kit 4, a sample stage 51, an adjustment mechanism 52, a laser light source 53, a condensing lens 54, an illuminating light source 55, an objective lens 56, a camera 57, a spectroscope 58, an optical component 59, and a controller 6. Optical component 59 includes a mirror 591, a half mirror 592, and a lens 593.

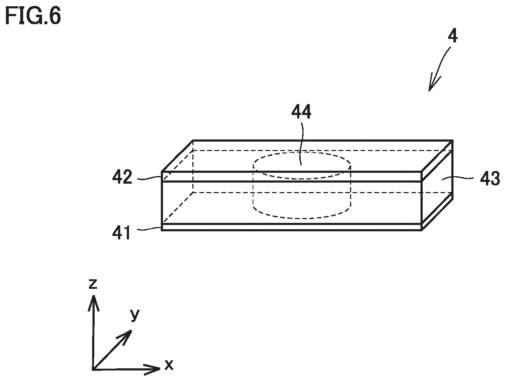

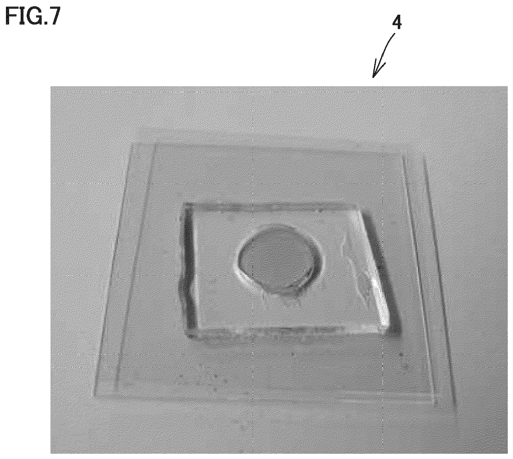

Detection kit 4 retains a sample. The sample is a liquid sample that may contain target DNA 9. The liquid (dispersion medium) may be of any type. In this example, the liquid is water. Probe particles 1 and probe particles 2 are introduced into the sample. Figs. 6 and 7 describe the configuration example of detection kit 4.

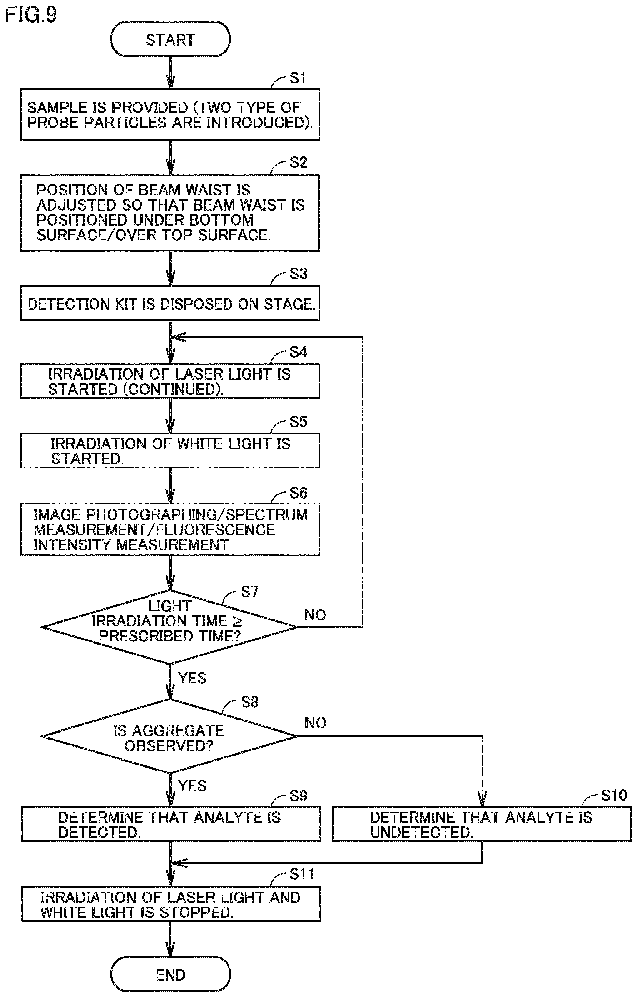

Sample stage 51 is configured to hold detection kit 4. Although other detection kits are not shown, multiple detection kits 4 may be provided. In the case, multiple detection kits 4 are disposed on sample stage 51 sequentially, followed by the detection processing described below (refer to Fig. 9).

Adjustment mechanism 52 is a mechanism for adjusting, for example, a three-axis (x-axis, y-axis, and z-axis) stage. Adjustment mechanism 52 adjusts the position of sample stage 51 in horizontal directions and the height thereof in the vertical direction in accordance with commands from controller 6. This enables adjusting the relative position relationship between detection kit 4 and condensing lens 54 and adjusting the relative position relationship between detection kit 4 and objective lens 56. Instead of sample stage 51, condensing lens 54 and/or objective lens 56 may be provided with adjustment mechanism 52, or not only sample stage 51 but also condensing lens 54 and/or objective lens 56 may be provided with adjustment mechanism 52. Alternatively, not only sample stage 51 but also condensing lens 54 and/or objective lens 56 may be provided with adjustment mechanism 52.

Laser light source 53 emits laser light (indicated with L1) as continuous waves (CWs) in accordance with commands from controller 6. The laser light emitted from laser light source 53 is reflected on mirror 591 to travel to condensing lens 54. The laser light has a wavelength outside a wavelength region of electronic resonance of probe particles 1 (metal nanoparticles 11 are subjected to localized surface plasmon resonance) and outside a wavelength region of electronic resonance of probe particles 2(microparticles 21 are subjected to exciton resonance). In the present embodiment, the laser light has a wavelength in a near-infrared region (for example, 1064 nm). The laser light corresponds to "non-resonant light" according to the present disclosure. Laser light source 53 corresponds to the "light source" according to the present disclosure.

Condensing lens 54 condenses the laser light reflected on mirror 591. Condensing lens 54 is typically an objective lens provided on a microscope. Condensing lens 54 is particularly preferably an objective lens with a correction collar. Detection kit 4 is irradiated with the laser light condensed by condensing lens 54.

Illuminating light source 55 irradiates white light for illuminating the sample on detection kit 4 (indicated with L2). For example, a halogen lamp or a mercury lamp is usable as illuminating light source 55. In the present embodiment, a mercury lamp is used in combination with a fluorescent filter (Standard series FITC Basic C-FL, which is available from NIKON CORPORATION). The white light emitted from illuminating light source 55 is transmitted through detection kit 4.

Objective lens 56 captures the white light transmitted through detection kit 4. Some of the white light introduced into objective lens 56 is transmitted through half mirror 592. The other white light is reflected on half mirror 592. The transmitted white light is condensed by lens 593, and the condensed light is guided into camera 57. Meanwhile, the reflected white light is guided into spectroscope 58.

Camera 57 is, for example, a light receiver including a CCD (charge-coupled device) image sensor or a CMOS (complementary metal oxide semiconductor) image sensor. Camera 57 photographs the sample on detection kit 4 in accordance with commands from controller 6 to output a signal indicating the photographed image to controller 6. An image to be photographed with camera 57 may be a still image or a movie.

Spectroscope 58 is a light receiver capable of light dispersion in the region from ultraviolet to near-infrared. Spectroscope 58 measures spectral characteristics of the sample on detection kit 4 to output a signal indicating the measured spectral characteristics to controller 6. More specifically, spectroscope 58 measures the absorbance spectrum or the fluorescence intensity of aggregate 3 formed in the sample on detection kit 4 to output a signal indicating the measurement results to controller 6. Spectroscope 58 in the case of the fluorescence observation may be a fluorescent spectral detector.

Controller 6 includes a processor 61 such as a CPU (central processing unit); memory 62 such as ROM (read only memory) or RAM (random access memory); and an input/output port 63 through which various signals are input/output. Controller 6 controls the devices in detection system 100 (adjustment mechanism 52, laser light source 53, illuminating light source 55, camera 57, and spectroscope 58). Moreover, controller 6 detects target DNA 9 in the sample based on the image photographed with camera 57. Controller 6 detects target DNA 9 in the sample based on the absorbance spectrum or the fluorescence intensity measured with spectroscope 58. The results of these detection processings will be described below.

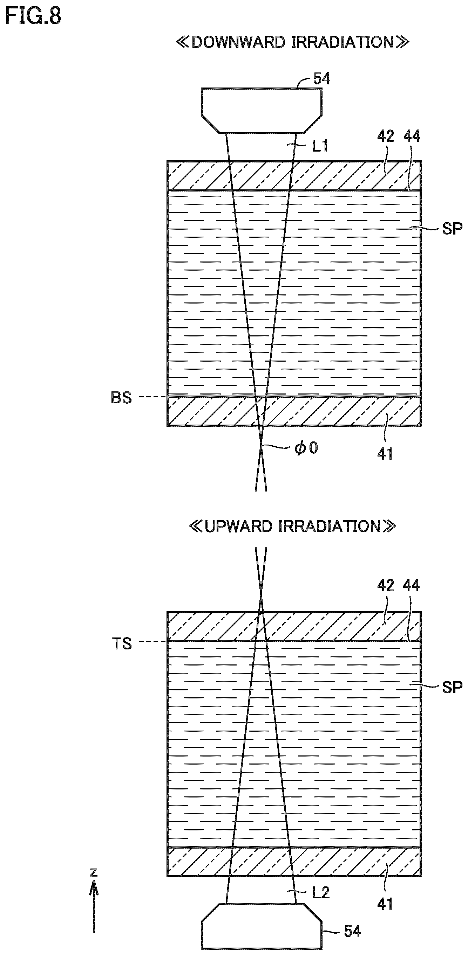

The optical system for photographing or measurement shown in Fig. 4 (illuminating light source 55, objective lens 56, half mirror 592, and lens 593) is an example. For example, the optical system of detection system 100 shown in Fig. 4 is configured so that a sample is irradiated with white light emitted downward from illuminating light source 55 (refer to the downward irradiation in Fig. 8), and camera 57 photographs the sample positioned above camera 57 (transmission mode of an inverted microscope). The optical system of detection system 100 may however be configured so that a sample is irradiated with white light emitted upward from illuminating light source 55 (refer to the upward irradiation in Fig. 8), and camera 57 photographs the sample positioned below camera 57 (transmission mode of an upright microscope). Spectroscope 58 is also disposed in the same way. The optical system of detection system 100 may include other optical components (for example, a mirror, a dichroic mirror, a beam splitter, a filter, and an optical fiber) instead of or in addition to optical component 59 shown in Fig. 4.

The function of the optical system shown in Fig. 4 can also be achieved with the optical system of an upright microscope (upright style). Hereinafter, the more detailed configuration of the upright style will be described.

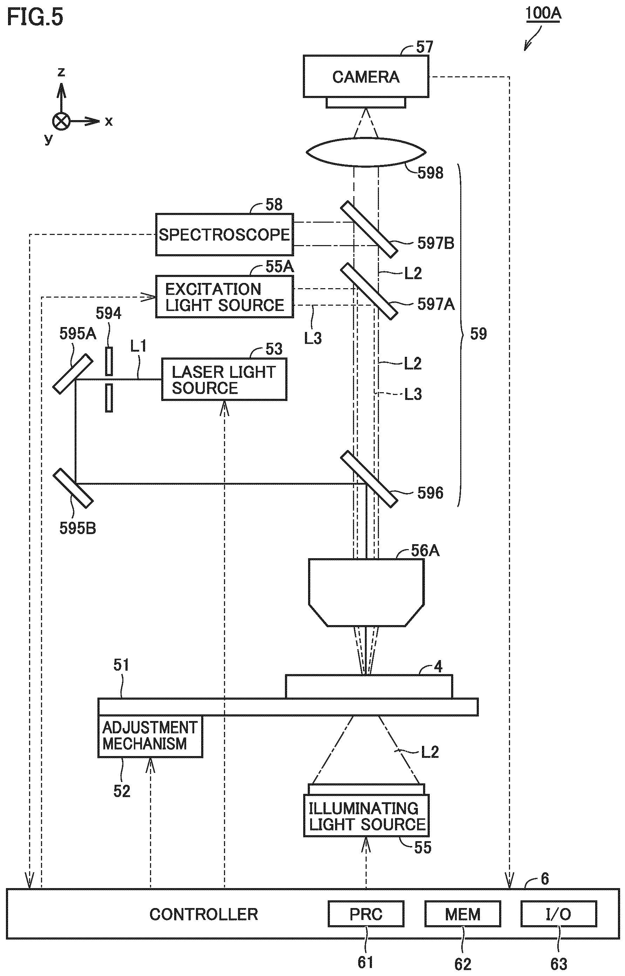

Fig. 5 shows another example of the entire configuration of a system for detecting an analyte in the upright configuration according to the present embodiment. A detection system 100A comprises a detection kit 4, a sample stage 51, an adjustment mechanism 52, a laser light source 53, an illuminating light source 55, an excitation light source 55A, an objective lens 56A, a camera 57, a spectroscope 58, an optical component 59, and a controller 6. For example, optical component 59 includes a shutter 594, mirrors 595A and 595B, a dichroic mirror 596, beam splitters 597A and 597B, and a lens 598.

Laser light (indicated with L1) from laser light source 53 passes through shutter 594, and is reflected on mirrors 595A and 595B and dichroic mirror 596 to travel to objective lens 56A. In the optical system shown in Fig. 5, objective lens 56A has both functions of condensing lens 54 and objective lens 56 in Fig. 4. The laser light is condensed with objective lens 56A, and detection kit 4 is irradiated with the condensed laser light emitted downward.

Fig. 5 also shows an excitation light source 55A (for example, a mercury lamp) for emitting excitation light (indicated with L3) for fluorescence observation. The excitation light is reflected on beam splitter 597A to be transmitted through dichroic mirror 596. The excitation light is condensed with objective lens 56A, and detection kit 4 is irradiated with the condensed excitation light emitted downward. A filter cube not shown and adapted to the color (wavelength) of the fluorescence can be disposed above objective lens 56A at the time of the fluorescence observation.

Detection kit 4 is irradiated with white light (indicated with L2) emitted upward from illuminating light source 55. The white light passed through detection kit 4 is transmitted through dichroic mirror 596 and beam splitter 597A. Some of the white light transmitted through beam splitter 597A is further transmitted through beam splitter 597B and condensed with lens 598 to be guided into camera 57. The other white light transmitted through beam splitter 597A is reflected on beam splitter 597B to be guided into spectroscope 58. Since the other configuration shown in Fig. 5 are the same as the corresponding configuration shown in Fig. 4, the detailed description thereof is not repeated.

Fig. 6 is a perspective view showing the configuration of detection kit 4 schematically. Fig. 7 shows an image of actually manufactured detection kit 4. With reference to Figs. 6 and 7, detection kit 4 includes a substrate 41, a cover 42, and a spacer 43.

The materials of substrate 41, cover 42, and spacer 43 are transparent to both laser light and white light. Such materials are, for example, glass, quartz, and silicone. In this example, both of substrate 41 and cover 42 are cover glasses. The cover glasses have a size of 32 mm in length × 24 mm in width × 0.17 mm in height. Spacer 43 is disposed between substrate 41 and cover 42. In this example, spacer 43 is double-sided adhesive tape. The double-sided adhesive tape has a size smaller than substrate 41 and cover 42 (both are cover glasses). A columnar well 44 (with a diameter of 6 mm) is provided at the center of the double-sided adhesive tape as an inner space for retaining the sample.

### <Upward irradiation and downward irradiation>

Fig. 8 shows diagrams for describing aspects in which detection kit 4 is irradiated with laser light. Fig. 8 shows how the sample (indicated with SP) retained in well 44 is irradiated with the laser light condensed with condensing lens 54. The beam waist of the laser light is formed at the focal point of condensing lens 54. The beam diameter at the beam waist (minimum spot diameter ϕ0) is, for example, from several micrometers to several dozen micrometers.

In the present embodiment, the sample is irradiated with the laser light in one aspect of the following two irradiation aspects. An aspect for irradiating laser light in which detection kit 4 is irradiated with the laser light emitted downward with condensing lens 54 disposed above detection kit 4 is referred to as "downward irradiation". On the contrary, an aspect for irradiating laser light in which detection kit 4 is irradiated with the laser light emitted upward with condensing lens 54 disposed below detection kit 4 is referred to as "upward irradiation". The downward irradiation can be achieved in the upright configuration as shown in Fig. 5. The upward irradiation can be achieved with the optical system as shown in Fig. 5 turned upside down, with the optical system of the inverted microscope in which detection kit 4 is irradiated with the laser light from laser light source 53 through objective lens 56A below sample stage 51 (in the inverted style).

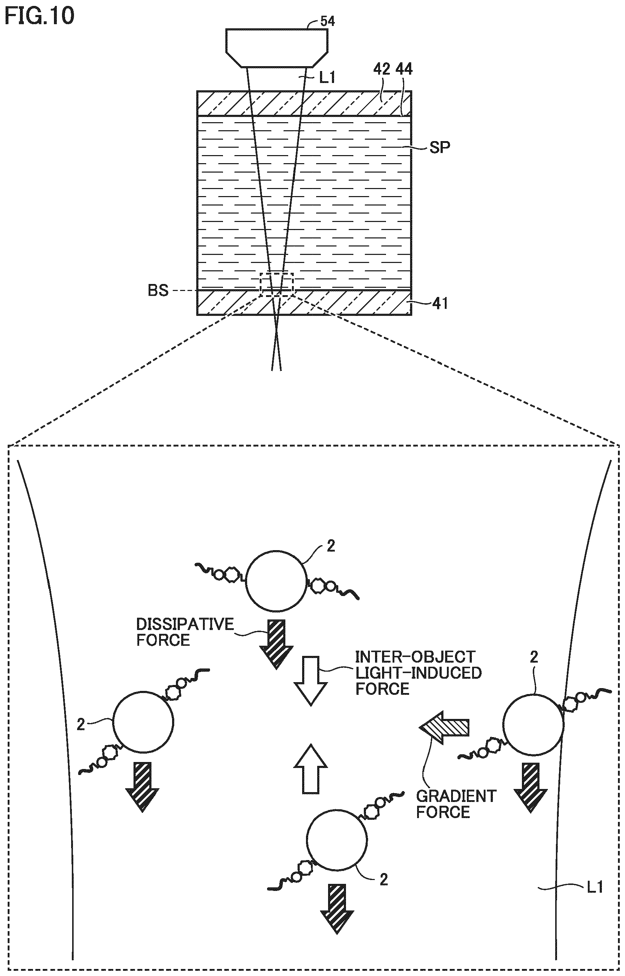

The beam waist in the downward irradiation is preferably positioned under bottom surface BS of well 44 in the vertical direction. The beam waist in the upward irradiation is preferably positioned over top surface TS of well 44 in the vertical direction. That is, the condition that the beam waist is away from top surface TS or bottom surface BS of well 44, in other words, the condition that the laser light is positioned behind detection kit 4 in its propagation direction, is preferably satisfied. Hereinafter this condition will be described as a "defocusing condition". The defocusing condition will be described in Fig. 10.

### <Processing flow>

Fig. 9 is a flow chart showing a processing procedure for subjecting target DNA 9 to detection processing. If a predetermined condition is satisfied (for example, if a user operates a start button (not shown)), this flow chart is executed. The steps are basically achieved by the software processing of controller 6. Some or all thereof may be achieved by hardware (electric circuits) manufactured in controller 6. Hereinafter, a step is abbreviated as "S".

In S1, controller 6 prepares the sample. More specifically, controller 6 introduces probe particles 1 and 2 into the sample that may contain target DNA 9. For example, controller 6 controls dispensers (not shown) to drop a dispersion of probe particles 1 and a dispersion of probe particles 2 prepared at predetermined concentrations, respectively, into the sample. The user may introduce probe particles 1 and 2 into the sample manually.

In S2, controller 6 adjusts the height using adjustment mechanism 52 so that the beam waist of the laser light is at a desired position over top surface TS of well 44 (in the case of the upward irradiation) or at a desired position under bottom surface BS of well 44 (in the case of the downward irradiation).

In general, all of substrate 41 and cover 42 (cover glasses), and spacer 43 (double-sided adhesive tape) can vary in thickness to a certain degree (for example, by several micrometers to several dozen micrometers). The height adjustment after the sample provision enables absorbing the variation in thickness. The height adjustment and the sample provision may be performed in any order. For example, if detection kits 4 are manufactured so highly precisely as to vary negligibly little in thickness, the processing S1 and the processing S2 may be performed in random order.

In S3, controller 6 disposes detection kit 4 on sample stage 51. This processing can be achieved, for example, with a mechanism for conveying detection kit 4 (not shown). The user may dispose detection kit 4 manually.

In S4, controller 6 controls laser light source 53 to start (or continue) irradiating detection kit 4 with laser light. If the sample contains target DNA 9, this binds probe particles 1 and 2 to target DNA 9 (results in hybridization) to form aggregate 3 of probe particles 1 and 2 (refer to Fig. 3). The formed aggregate 3 grows with time for irradiating the laser light. The formation and the growth of aggregate 3 are promoted during the laser light irradiation in accordance with mechanisms to be described below in Figs. 10 to 13.

In S5, controller 6 controls illuminating light source 55 to start irradiating detection kit 4 with white light.

In S6, controller 6 controls camera 57 to photograph an image of detection kit 4 at a laser spot (position irradiated with laser light). Alternatively, controller 6 controls spectroscope 58 to measure the absorbance spectrum or the fluorescence intensity of detection kit 4 at the laser spot. Controller 6 may perform both the above-mentioned measurements simultaneously.

In S7, controller 6 determines whether time has elapsed from the start of the laser light irradiation reaches prescribed time (for example, 180 seconds or 240 seconds in Examples, described below). If the light irradiation time does not reach the prescribed time (NO in S7), controller 6 returns the processing to S4. This continues irradiating laser light and white light, photographing the image, and measuring the spectrum. If the light irradiation time has reached the prescribed time (YES in S7), controller 6 advances the processing to S8.

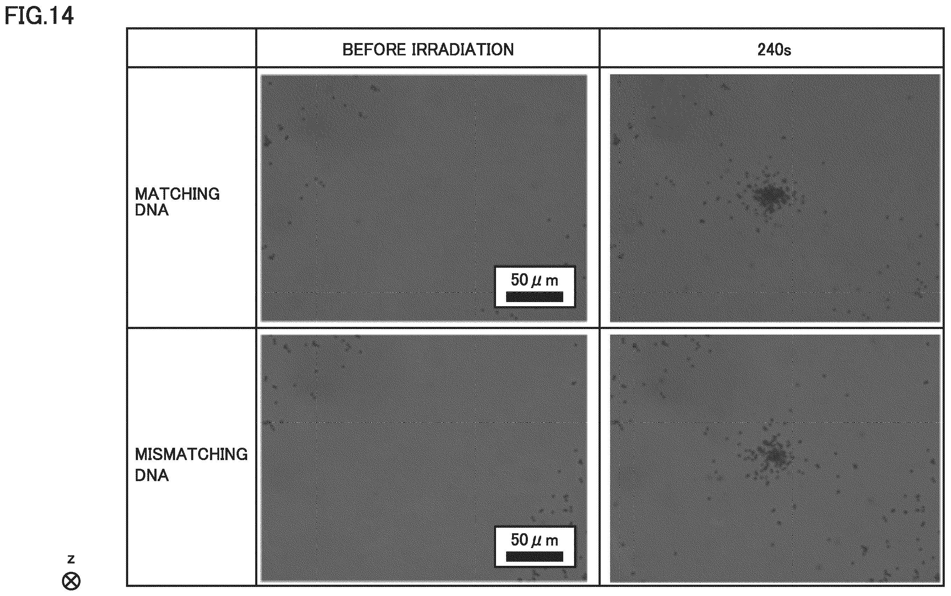

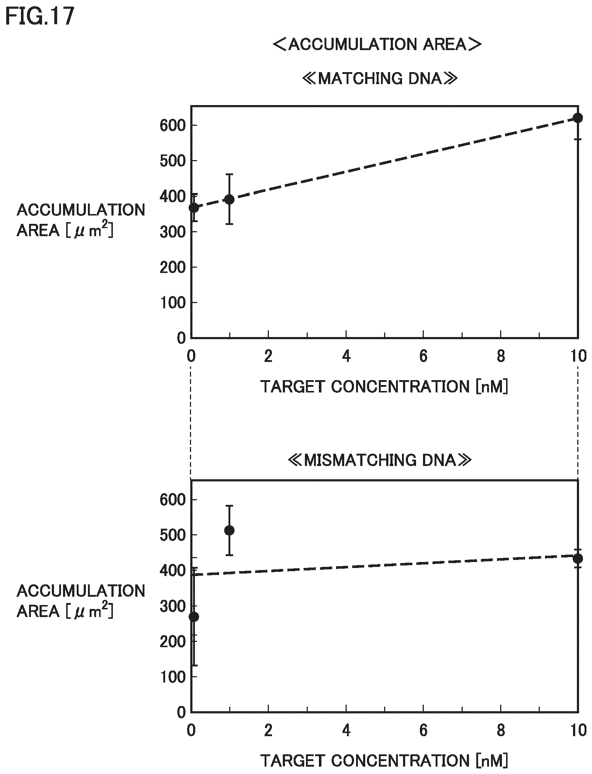

In S8, controller 6 subjects the photographed image to predetermined image processing to determine whether aggregate 3 of probe particles 1 and 2 is observed in the image. For example, if the size of aggregate 3 (accumulation area of probe particles 1 and 2 described below) exceeds a standard area, controller 6 can determine that aggregate 3 is observed (refer to Figs. 14 and 17).

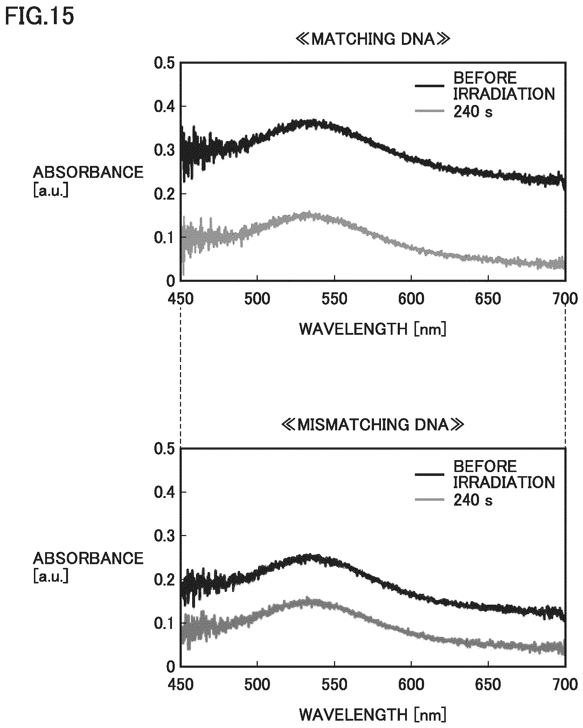

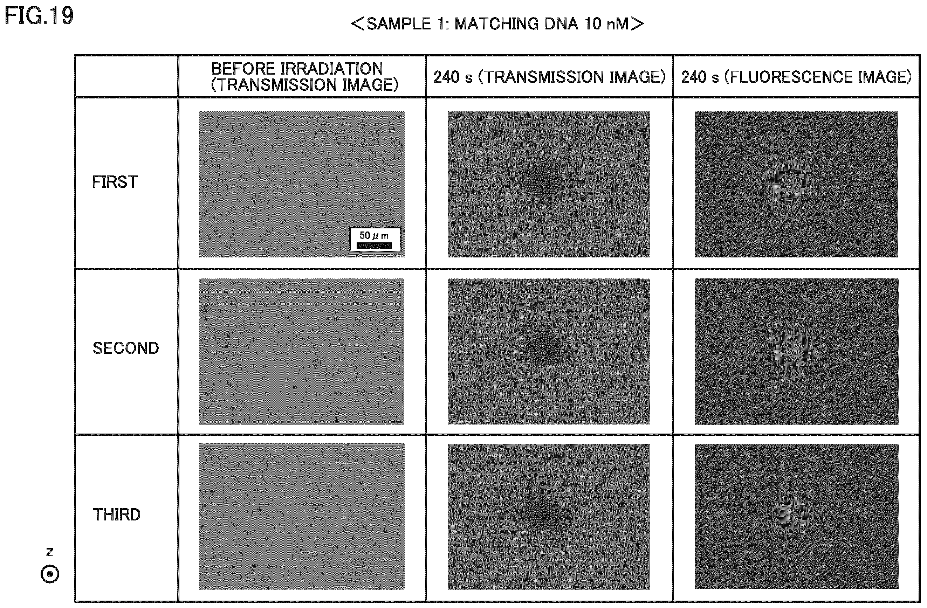

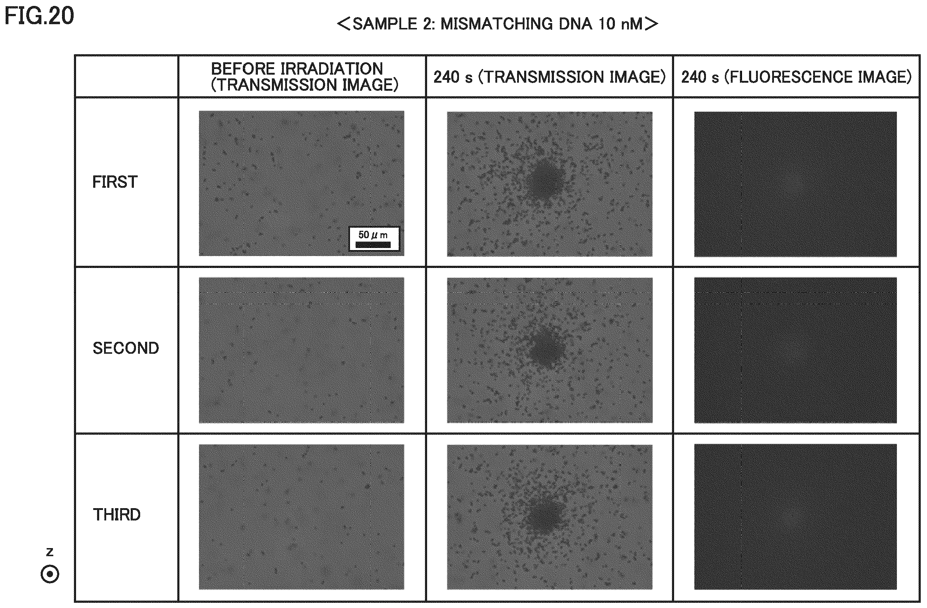

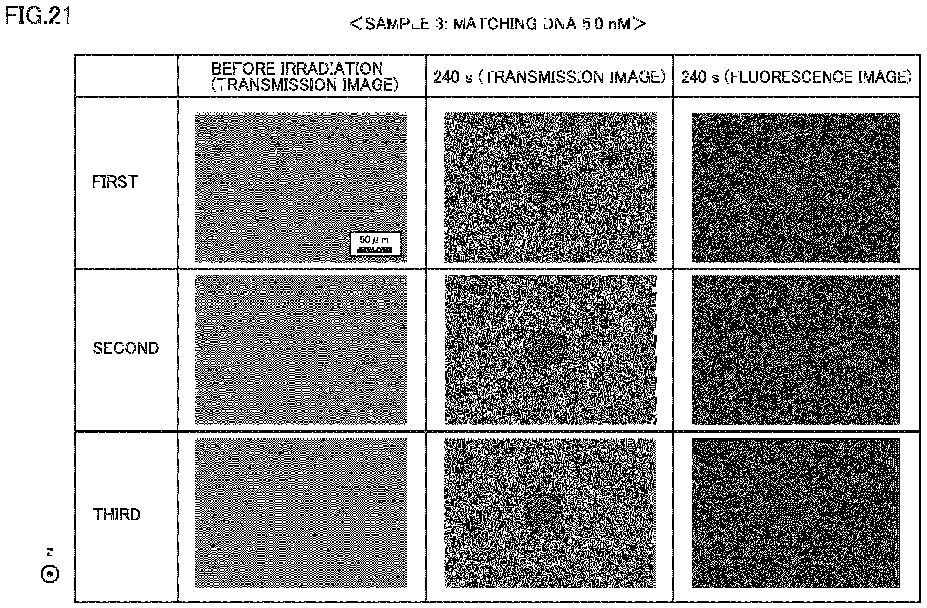

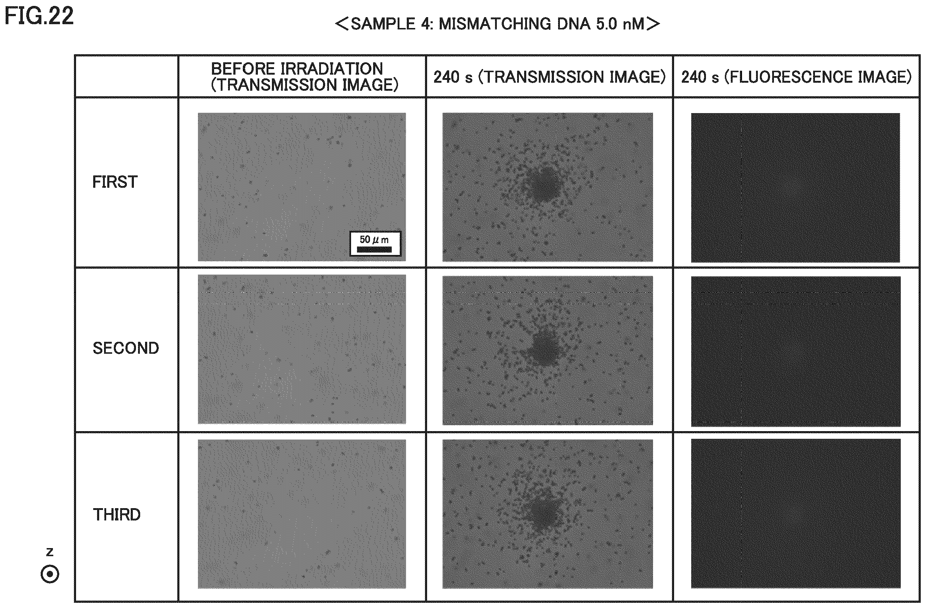

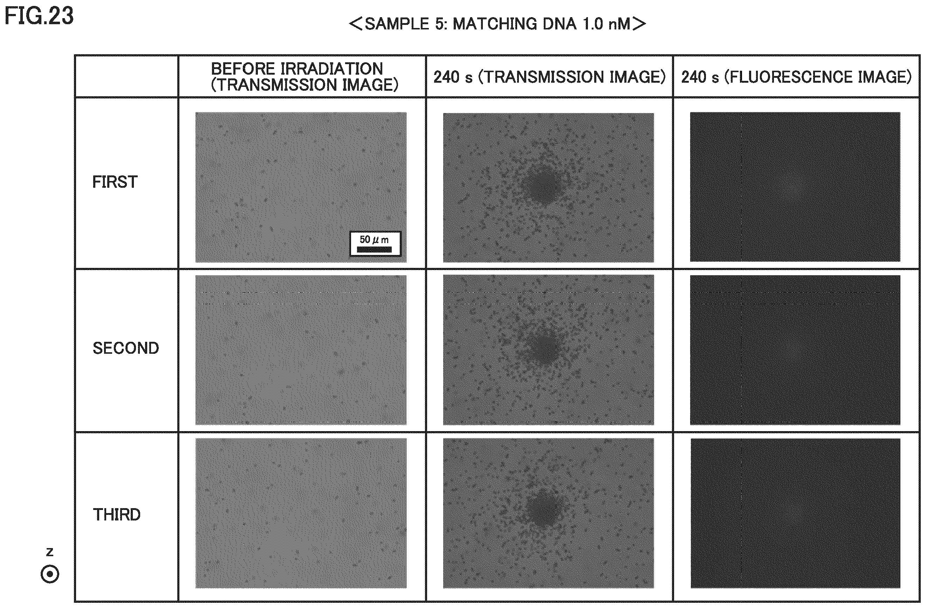

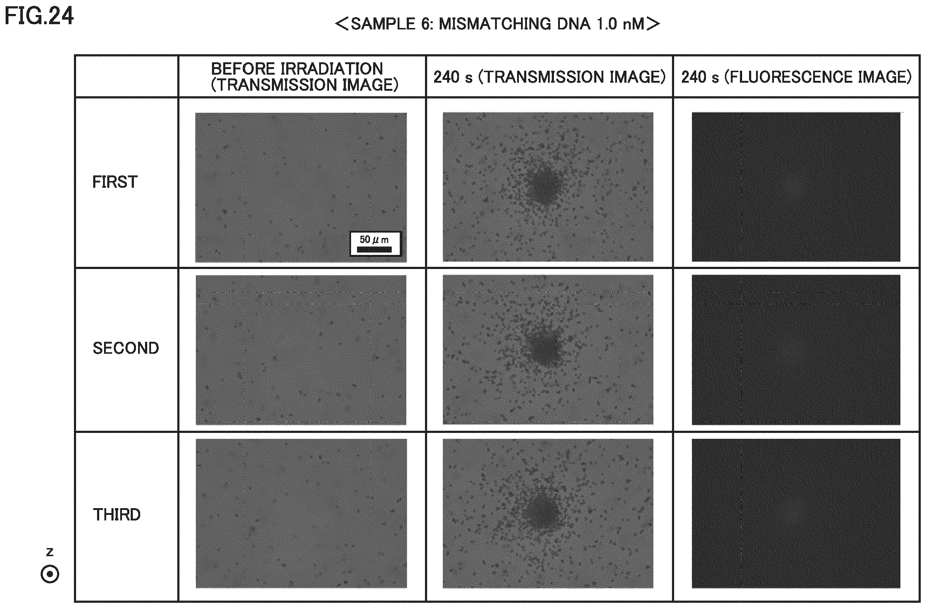

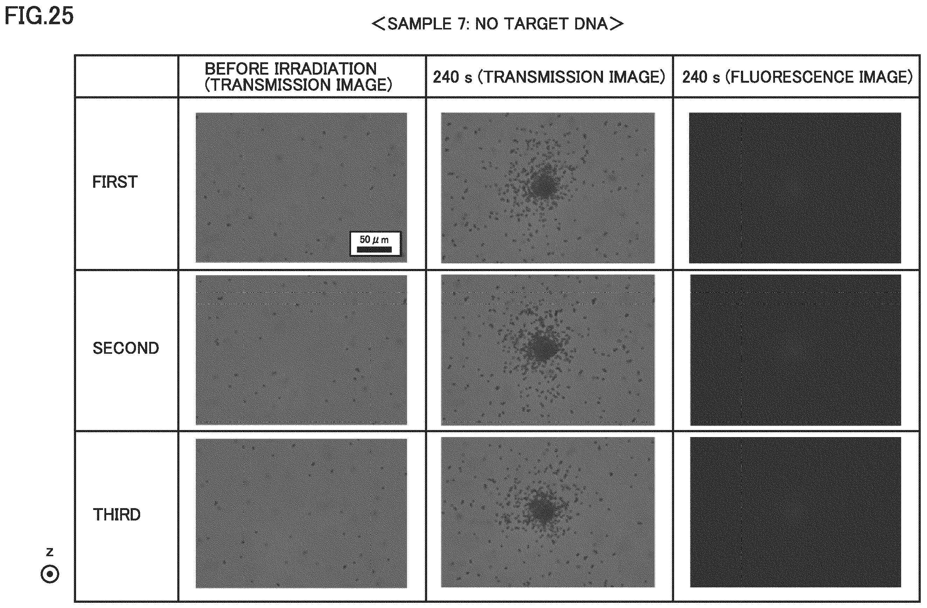

Alternatively, controller 6 may analyze the measured absorbance spectrum or fluorescence intensity to determine whether aggregate 3 is present. For example, if the difference between the absorbances before and after the light irradiation or between the fluorescence intensities before and after the light irradiation exceeds a predetermined amount, controller 6 may determine that aggregate 3 is observed (refer to Figs. 15, 16, and 26). For example, if, in the image or fluorescence image after the light irradiation, the absorbance difference or the fluorescence intensity difference between the light irradiation region and the background region (region sufficiently away from the irradiation region) exceeds a predetermined amount, controller 6 may determine that aggregate 3 is observed (refer to, for example, Figs. 19 to 25, Fig. 28, Fig. 31, and Fig. 36). Controller 6 thus detects aggregate 3 based on a variation in absorbance or fluorescence intensity accompanying light irradiation. The variation may be typically a difference, but the variation may be, for example, a ratio.

Since many fluorescent probes are bleached, the difference between the fluorescence intensities before and after the light irradiation can include the intensity change due to the bleach of fluorescent probes. Accordingly, the difference between the fluorescence intensity in the light irradiation region and the fluorescence intensity in the background region in the fluorescence image after the light irradiation is desirably used for accurate comparison especially in fluorescence measurement. Meanwhile, some fluorescent probes (for example, nanodiamonds) are scarcely bleached. If fluorescent probes to be scarcely bleached are used, the difference between the fluorescence intensities in the identical region before and after the light irradiation may be used.

If aggregate 3 of probe particles 1 and 2 is observed (YES in S8), controller 6 determines that the sample contains target DNA 9 (S9). Meanwhile, if aggregate 3 is not observed (NO in S8), controller 6 determines that target DNA 9 is undetected (not contained in the sample) (S10).

In S11, controller 6 controls laser light source 53 to stop irradiating laser light, and controls illuminating light source 55 to stop irradiating white light. This stops a series of processings.

### <Mechanism>

As described in Fig. 3, probe particles 1 and 2 encounter target DNA 9, resulting in repeated hybridization, to form aggregate 3 of probe particles 1 and 2 through target DNA 9. As aggregate 3 grows, probe particles 1 and 2 and target DNA 9 around aggregate 3 are more likely to encounter aggregate 3. The frequency of the hybridization consequently increases at an accelerated pace, resulting in the promotion of the growth of aggregate 3. The present embodiment thus enables achieving the "light-induced acceleration" in which the laser light irradiation accelerates the aggregation of probe particles 1 and 2. The light-induced acceleration is achieved by mechanisms related to dissipative force, thermal convection, and Brownian motion.

### <<Dissipative force>>

Fig. 10 is an illustration for describing dissipative force acting on probe particles 1 and 2. Fig. 10 illustrates the downward irradiation.

If detection kit 4 is irradiated with laser light, light-induced force acts on probe particles 1 and 2 in the sample. In more detail, in addition to inter-object light-induced force and gradient force acting on probe particles 1 and 2, dissipative force acts on probe particles 1 and 2 in the same direction as the laser light irradiation direction. In the case of the downward irradiation, dissipative force acts downward on probe particles 1 and 2 to press probe particles 1 and 2 against bottom surface BS of well 44. Although not shown in the figure, in the case of the upward irradiation, dissipative force acts upward on probe particles 1 and 2 to press probe particles 1 and 2 against top surface TS of well 44.

In general, if a substance to be irradiated has a size (diameter) smaller than the wavelength of light under non-resonant conditions (if the substance is nanoparticles in the present embodiment), gradient force among the components of light-induced force is proportional to the volume of the substance, and dissipative force is proportional to the square of the volume of the substance. Meanwhile, if a substance to be irradiated has a size equivalent to the wavelength of light (if the substance is microparticles), the light causes Mie scattering. Dissipative force generated by the Mie scattering is proportional to the square of the diameter of the substance.

In the present embodiment, probe particles 2 (microparticles 21) have a diameter of 2 µm, and the laser light has a wavelength of 1064 nm. That is, the diameter of probe particles 2 is equivalent to the wavelength of the laser light. Accordingly, the laser light with which detection kit 4 is irradiated generates dissipative force acting on probe particles 2 due to Mie scattering. The diameter of probe particles 2 (2 µm) is around 100 times larger than the diameter of probe particles 1 (30 nm). Accordingly, dissipative force acting on probe particles 2 is remarkably strong as compared with dissipative force acting on probe particles 1. Specifically, if laser light at an intensity of around 100 mW is irradiated, dissipative force that the gold nanoparticles receive is around several dozen fN (femtonewtons), and dissipative force that the microparticles receive is meanwhile around several dozen pN (piconewtons) that is three orders of magnitude larger than that of the gold nanoparticles.

Moreover, in the case of the downward irradiation, the dissipative force due to Mie scattering enables strongly pressing probe particles 2 against bottom surface BS of well 44. In that case, the density of probe particles 2 on bottom surface BS of well 44 is locally high as compared with the density of probe particles 2 at other positions (positions sufficiently away from the beam waist). Then, a probability of probe particles 2 encountering target DNA 9 becomes higher near bottom surface BS of well 44. If probe particles 2 encounter target DNA 9, as described in Fig. 3, host molecules 22 (probe DNA) modifying the surfaces of probe particles 2 are hybridized with target DNA 9.

In the present embodiment, in the case of downward irradiation, the beam waist is furthermore positioned under bottom surface BS of well 44. If the beam waist is intentionally positioned out of bottom surface BS of well 44 (defocused), the area on bottom surface BS of well 44 irradiated with the laser light is larger than if the beam waist is positioned on bottom surface BS of well 44 (focused). By securing the irradiated area having a size approximate to a certain size, probe particles 2 are widely pressed against bottom surface BS. Accordingly, the probability of probe particles 2 encountering target DNA 9 can be made even higher.

The irradiation of probe particles 2 having a size in the micrometer order with laser light having a wavelength equivalent to the size can locally increase the density of probe particles 2 on bottom surface BS to hybridize probe particles 2 with target DNA 9 with high efficiency. Although detailed description is not repeated, in the case of the upward irradiation, the irradiation also results in hybridization in the same way.

### <<Thermal convection>>

Probe particles 1 contain metal nanoparticles 11. If probe particles 1 are irradiated with laser light, the free elections of metal nanoparticles 11 form localized surface plasmons, and are oscillated with laser light. This leads to polarization. This polarization energy is converted into lattice oscillation energy by the Coulomb interaction between the free electrons and the atomic nuclei. Metal nanoparticles 11 consequently generate heat (photothermal effect). The photothermal effect causes thermal gradient in the sample. This steadily leads to regular thermal convection (buoyant convection and/or Marangoni convection) toward the beam waist. New probe particles 1 and 2 and target DNA 9 are conveyed to the beam waist by this thermal convection. In that case, a probability of probe particles 1 encountering target DNA 9 is increased, and a probability of probe particles 2 encountering target DNA 9 is increased compared to in static liquid.

The irradiation of probe particles 1 containing metal nanoparticles 11 with the laser light leads to thermal convection toward the beam waist.to hybridize probe particles 1 and 2 with target DNA 9 with still higher efficiency.

### <<Brownian motion>>

Probe particles 1 are more greatly influenced by Brownian motion (random diffusion motion due to thermal fluctuation in water) than probe particles 2. The results of estimating the Brownian motion of the particles from the diffusion distance and the average interparticle distance will be described.

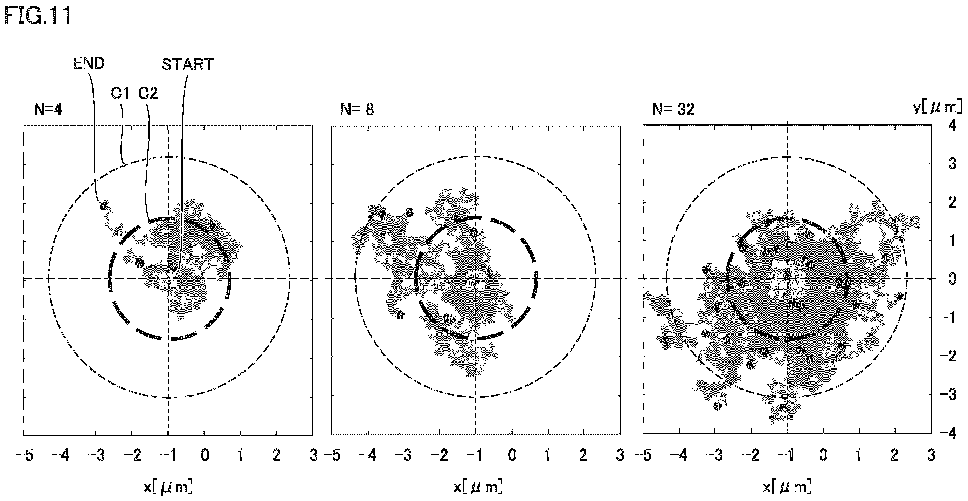

Fig. 11 shows the results of simulating the Brownian motion of probe particles 1. Fig. 11 shows the trajectories drawn by probe particles 1 for 0.1 seconds if N probe particles 1 are disposed in water. The simulations were performed in the three cases where N = 4, 8, and 32, respectively. The diameter of probe particles 1 was set at 40 nm, and the temperature of water is set at 25°C. The initial positions of probe particles 1 were at the centers of the images, indicated with START. The final positions of probe particles 1 were at positions indicated with END.

The average diffusion radii σ of fine particles after t seconds are calculated in accordance with the following expression (1). The average diffusion radii σ are positioned between the circles C1 and C2 in the figure.
[Math. 1] $σ = \sqrt{\left〈{\left({x}_{i}^{B}\right)}^{2}\right〉} = \sqrt{\left〈{\left({y}_{i}^{B}\right)}^{2}\right〉} = \sqrt{\left〈{\left({z}_{i}^{B}\right)}^{2}\right〉} = \sqrt{\frac{2 kT}{ξ} t}$

k is Boltzmann's constant, and T is the absolute temperature of water. ξ is a coefficient of friction, and is expressed as ξ = 6πηa using the viscosity coefficient of water η and the particle radius of water a. Expression (1) shows that the diffusion distances per unit time of the particles are inversely proportional to the square root of the size (radius or diameter) of the particles. For example, probe particles 1 having a diameter of 30 nm diffuses over a distance around eight times longer than probe particles 2 having a diameter of 2 µm.

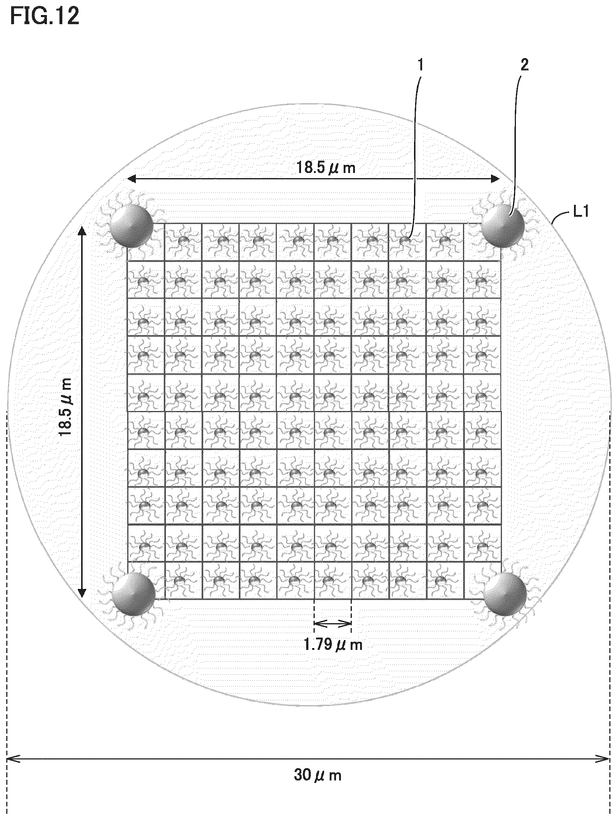

Fig. 12 is a conceptual figure for describing the average interparticle distances of probe particles 1 and 2. It was assumed that probe particles 1 were gold nanoparticles having a diameter of 30 nm, and probe particles 2 were polystyrene particles having diameter of 2 µm. It was assumed that the concentration of probe particles 1 was 1.73 × 10¹¹ [particles/mL], and the concentration of probe particles 2 was 1.58 × 10⁸ [particles/mL].

The average interparticle distance of probe particles 1 is calculated as 1.79 µm, and the average interparticle distance of probe particles 2 is calculated as 18.5 µm. In this case, around 100 probe particles 1 are present in a square wherein probe particle 2 are disposed at each of its four apexes under planar observation as shown in Fig. 12. In the figure, the circle surrounding all of probe particles 1 and 2 indicates a laser spot having a diameter of 30 µm. Under three-dimensional observation, around 1000 probe particles 1 are present in a cube wherein probe particle 2 is disposed at each of its eight apexes.

The diffusion distance per unit time of probe particles 1 is longer than that of probe particles 2. That is, probe particles 1 diffuse at higher speed than probe particles 2. While probe particles 1 repeat a high-speed random walk, probe particles 1 are therefore more likely to encounter target DNA 9. Accordingly, it is conceivable from a statistical viewpoint that target DNA 9 first encounters probe particles 1, and subsequently encounters probe particles 2 around probe particles 1. This means that the use of both probe particles 1 and 2 enables promoting especially the first hybridization of target DNA 9 compared to the use of only probe particles 2.

### <<Partial summary>>

Even though only probe particles 2 are used, strong dissipative force due to Mie scattering densifies probe particles 2 locally. This enables to increase the probability of probe particles 2 encountering target DNA 9 to promote the hybridization. The promotion effect is however exhibited to only a certain degree.

Meanwhile, in the case of the use of both probe particles 1 and 2, active Brownian motion of probe particles 1 provides an increased probability of probe particles 1 encountering target DNA 9. Moreover, the photothermal effect of probe particles 1 enables causing thermal convection to further increase the probability of probe particles 1 and 2 encountering target DNA 9. It is assumed that in Fig. 12, probe particles 2 are uniformly distributed in the entire sample. Since probe particles 2 receive strong dissipative force to be concentrated, the distance between probe particles 2 in the laser spot is however shorter. Accordingly, target DNA 9 hybridized with probe particles 1 has higher probability of encountering near probe particles 2 before target DNA 9 diffuses farther due to Brownian motion of probe particles 1. According to the present embodiment, such synergistic effect enables hybridizing probe particles 1 and 2 with target DNA 9 with very high efficiency.

As mentioned above, in the present embodiment, a "hetero-probe method" using probe particles 1 and 2 of different type having different sizes and made of different materials is adopted. This enables to increase the probability of probe particles 1 and 2 encountering target DNA 9 compared to the use of only one type of probe particles 1 and 2. The encounter enables highly efficient hybridization, resulting in enhancing light-induced acceleration further. Even though only a very small amount of target DNA 9 is present, the enhanced light-induced acceleration results in the formation and the growth of aggregate 3 in which probe particles 1 and 2 are highly densely aggregated within a short time. The present embodiment therefore enables detecting target DNA 9 with high sensitivity rapidly.

### [Example 1]

The results of evaluating influence that the combination of probe particles has on sensitivity for detecting target DNA will be described.

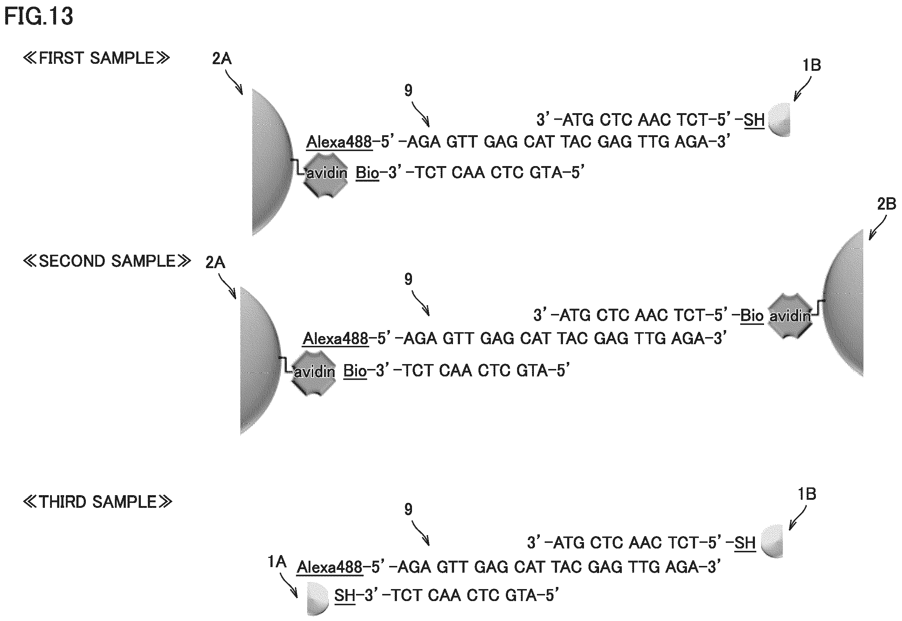

Fig. 13 illustrates describing the evaluation results of the combination of probe particles. Target DNA 9 labeled with fluorescent dye and having a sequence in which 24 bases were randomly arranged was used. Probe particles 1 having the base sequence completely complementary to the 12 bases at the 5'-end of target DNA 9 are described as "probe particles 1A". Meanwhile, probe particles 1 having the base sequence completely complementary to the 12 bases at the 3'-end of target DNA 9 are described as "probe particles 1B". The same applies to probe particles 2.

In this Example, first to third samples were prepared. The first sample contains probe particles 2A and 1B. The second sample contains probe particles 2A and 2B. The third sample contains probe particles 1A and 1B. The samples were measured for fluorescence intensity if target DNA 9 was contained and fluorescence intensity if target DNA 9 was not contained. The difference between the two fluorescence intensities were calculated.

The differences between the fluorescence intensities in the first to third samples were 19.57, 9.07, and 1.06, respectively. That is, the difference between the fluorescence intensities in the first sample is remarkably great as compared with the difference between the fluorescence intensities in the second sample and the difference between the fluorescence intensities in the third sample. This shows that the introduction of different types of probe particles (both probe particles 1 and 2) into the sample enables improving the sensitivity for detecting the target DNA 9 as compared with the introduction of the same type of probe particles (either of probe particles 1 and 2) into the sample.

### [Example 2]

The transmission images and the measurement results of the absorbance spectra in the case where target DNA not labeled with any fluorescent dye was used as an analyte will be described.

Two target DNAs 9 were prepared for comparison (control experiment). Since a first target DNA has a base sequence that is completely complementary to the host molecules (probe DNAs) of probe particles 1 and 2, the first target DNA hybridizes with probe particles 1 and 2. Hereinafter, such DNA is referred to as "matching DNA". Since a second target DNA has a base sequence that is not complementary at all to the host molecules (probe DNAs) of probe particles 1 and 2, the second target DNA does not hybridize with probe particles 1 and 2. Hereinafter, such DNA is referred to as "mismatching DNA".

In Example 2, A-T sequences, containing only adenine (A) and thymine (T), were used as the target DNAs 9. Specifically, host molecules 12 of probe particles 1 (gold nanoparticles) was 5'-TTT TTT TTT TTT-3'-(CH₂)₆-SH. Host molecules 22 of probe particles 2 (microparticles) was Biotin-3'-TTT TTT TTT TTT-5'. The matching DNA was 5'-AAA AAA AAA AAA AAA AAA AAA AAA-3'. The mismatching DNA was Biotin-5'-TTT TTT TTT TTT TTT TTT TTT TTT-3'.

The concentration of target DNA 9 (the matching DNA or the mismatching DNA) was 10 nM. Laser light was irradiated upward. The position of the beam waist of the laser light was adjusted so that the beam waist was positioned 45 µm above top surface TS of well 44. Condensing lens 54 had a magnification of 40. The laser light had an intensity of 525 mW (output after the laser light passed through detection kit 4). The light irradiation time was 240 seconds.

Fig. 14 shows an example of images photographed with camera 57 in Example 2. Fig. 15 shows an example of absorbance spectra measured with spectroscope 58 in Example 2. The abscissa axis indicates the wavelength, and the ordinate axis indicates the absorbance.

As shown in Fig. 14, if target DNA 9 was the matching DNA, the amount of probe particles 1 and 2 accumulated in the laser spot was larger than if target DNA 9 was the mismatching DNA. Moreover, as shown in Fig. 15, it was confirmed that the difference between the absorbance spectra before and after the irradiation was also greater in the case of the matching DNA than in the case of the mismatching DNA.

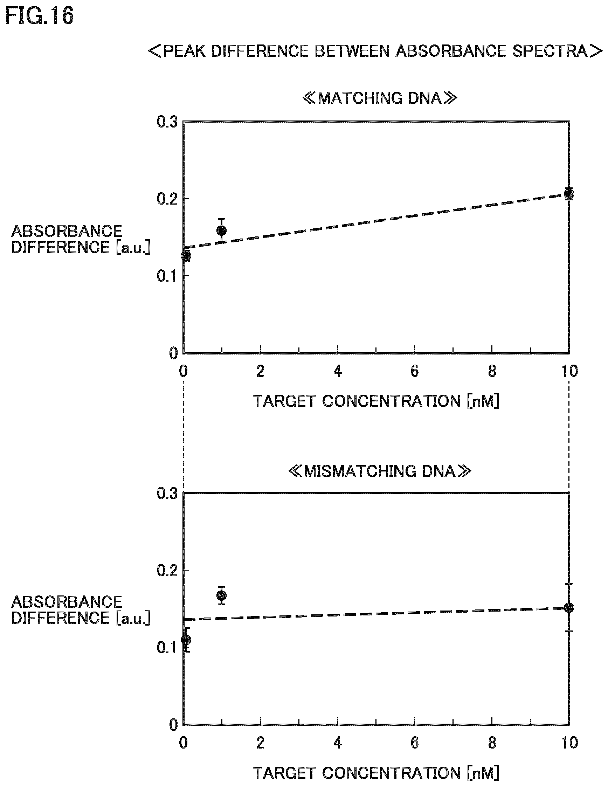

Fig. 16 shows the dependence of the absorbance differences on the target concentrations. The abscissa axis indicates the target concentration (concentration of target DNA 9), and the ordinate axis indicates the absorbance difference (difference between the peak values of the absorbance spectra before and after the irradiation).

The findings that the absorbance difference increased linearly with the target concentration increased regardless of whether target DNA 9 is matching DNA or mismatching DNA were obtained. If target DNA 9 was the matching DNA, the gradient of the straight line was 0.0071. If target DNA 9 was the mismatching DNA, the gradient of the straight line was 0.0019. That is, if target DNA 9 was the matching DNA, the absorbance difference increased with the target concentration more greatly than if target DNA 9 was the mismatching DNA.

Fig. 17 is figures showing the dependence of the accumulation area of probe particles 1 and 2 on the target concentration. The abscissa axis indicates the target concentration, and the ordinate axis indicates the accumulation area of probe particles 1 and 2. The accumulation area of probe particles 1 and 2 can be determined by calculating by image processing the area of a region having a color that becomes dark by the aggregation of probe particles 1 and 2.

The accumulation area increased linearly with the target concentration increase regardless of whether target DNA 9 was the matching DNA or the mismatching DNA. If target DNA 9 was the matching DNA, the gradient of the straight line was 25.16. If target DNA 9 was the mismatching DNA, the gradient of the straight line was 6.36. That is, if target DNA 9 was the matching DNA, the accumulation area increased with the target concentration more greatly than if target DNA 9 was the mismatching DNA.

### [Example 3]

The transmission images, the fluorescence images, and the measurement results of the fluorescence intensity in the case of using target DNA labeled with fluorescent dye as the analyte will be described.

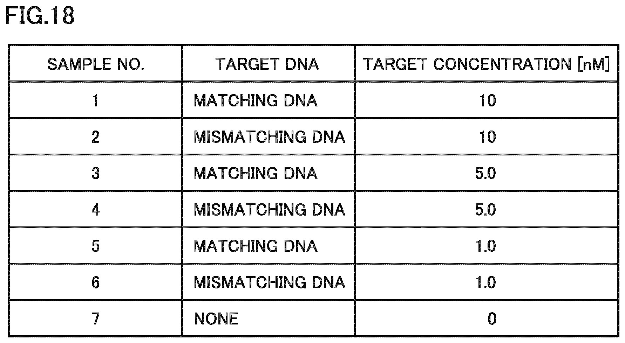

Fig. 18 is a table summarizing measurement concentrations in Example 3. Seven samples, samples 1 to 7, shown in Fig. 18 were prepared. The following conditions are common to all the samples 1 to 7. The volumes of the samples were 39 µL. Laser light was irradiated downward. The position of the beam waist of the laser light was adjusted so that the beam waist was positioned 45 µm under bottom surface BS of well 44. Condensing lens 54 had a magnification of 40. The intensity of the laser light was 640 mW. The light irradiation time was 240 seconds.

Figs. 19 to 25 are figures showing transmission images and fluorescence images of the samples 1 to 7, respectively. Even if a great difference was not observed depending on whether target DNA 9 was the matching or mismatching DNA in the transmission images at the left and the center, definite fluorescence was observed in the fluorescence images at the right only when target DNA 9 was the matching DNA.

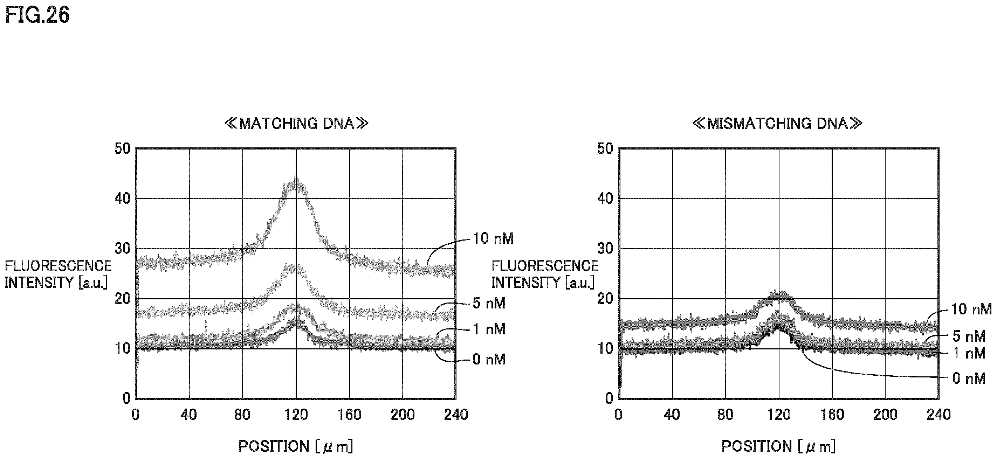

Fig. 26 shows an example of the measurement results of the dependence of the fluorescence intensity on the position. The abscissa axes indicate the measurement position of the fluorescence intensity along a principal surface direction (horizontal direction) of detection kit 4. The left ends of the transmission image and the fluorescence image correspond to 0 µm, and the right ends thereof correspond to 240 µm. The ordinate axes indicate the fluorescence intensity. Fig. 26 shows the average value of the measurement results of the fluorescence intensities of the three samples at each of four target concentrations. In Fig. 26, if target DNA 9 was matching DNA, the higher target concentration was, the higher fluorescence intensity was. Thus, it is found that the fluorescence intensity clearly depends on the target concentration even at a very low target concentration of 10 nM or less.

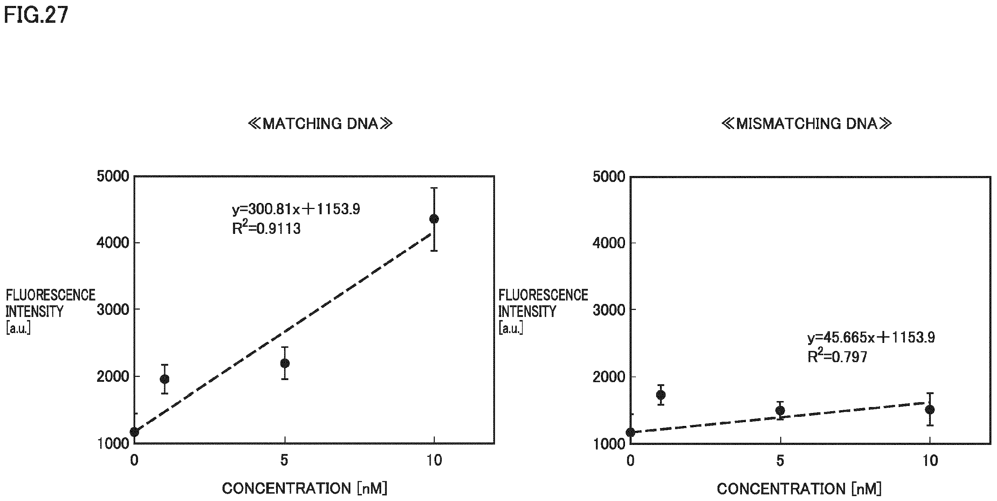

Fig. 27 shows an example of calibration curves acquired from the fluorescence intensities shown in Fig. 26. Fig. 27 shows calibration curves with respect to the concentrations of target DNA 9 in the case where target DNA 9 is the matching DNA (left graph) and in the case where target DNA 9 is the mismatching DNA (right graph), respectively. The background intensity is subtracted from the measured fluorescence intensity to obtain a value. The value is integrated in the position range of 105 to 135 µm in Fig. 26 to obtain each of these. The error bars indicate the standard deviations in the case of the number of measurements n = 3.

The linear calibration curves were obtained as shown in Fig. 27. This shows that even though the concentration of the matching DNA is less than 10 nM, the use of the calibration curve enables quantitatively evaluating the concentration of the matching DNA from the fluorescence intensity. If the concentration of the matching DNA is 10 nM, the concentration by mass of the matching DNA calculated from the molecular weight of the base pairs of the matching DNA is around 79.2 [fg/µL]. The detection limit calculated in the same way was a concentration by mass of around 7.92 to 39.6 [fg/µL].

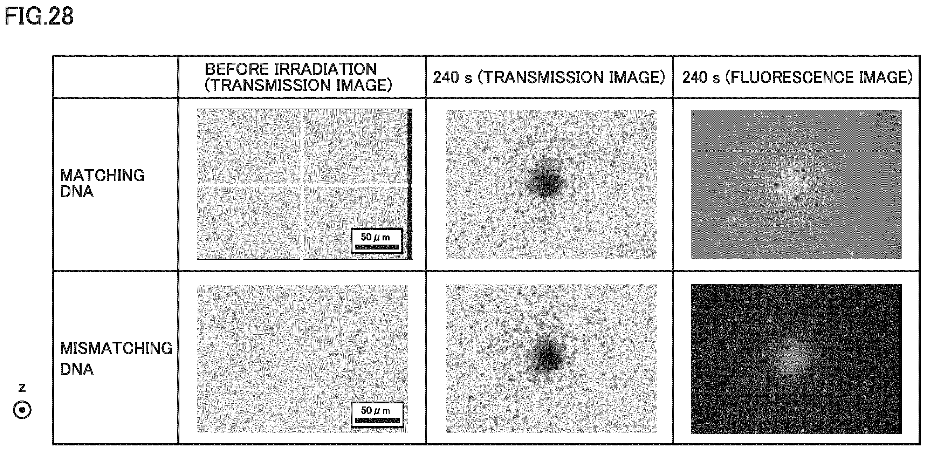

Fig. 28 shows images (transparent images and fluorescence images) in which it is photographed how fluorescence is enhanced on the surfaces of metal nanoparticles 11 (gold nanoparticles) contained in probe particles 1. Laser light was irradiated downward using condensing lens 54 provided in an upright microscope (not shown). The position of the beam waist of the laser light was adjusted so that the beam waist was positioned 45 µm under bottom surface BS of well 44. Condensing lens 54 had a magnification of 40. The laser light had an output of 640 mW. The light irradiation time was 240 seconds. The concentration of target DNA 9 was 10 nM. The concentration of probe particles 2 was 5.3 × 10⁸ [particles/mL].

The base sequence of target DNA 9 was a random sequence. The random sequence is a sequence containing all the four bases that are adenine (A), thymine (T), guanine (G), and cytosine (C). Specifically, host molecules 12 of probe particles 1 (gold nanoparticles) were 5'-ATG CTC AAC TCT-3'-(CH₂)₆SH (modified 3'-end). Host molecules 22 of probe particles 2 (microparticles) were Biotin-3'-TCT CAA CTC GTA-5' (modified 3'-end). A matching DNA was 5'-AGA GTT GAG CAT X TAC GAG TTG AGA-3'. The mismatching DNA was 5'-TCT CAA CTC GTA X ATG CTC AAC TCT-3'. X is the fluorescence dye (Alexa Flour 488, described above).

As shown in Fig. 28, if target DNA 9 was the matching DNA, strong fluorescence was observed not only at aggregate 3 of probe particles 1 and 2 but also around aggregate 3. Meanwhile, if target DNA 9 was the mismatching DNA, strong fluorescence around aggregate 3 was not observed.

Since target DNA 9 is labeled with the fluorescent dye, the formation of aggregate 3 by the hybridization of probe particles 1 and 2 with target DNA 9 makes metal nanoparticles 11 of probe particles 1 present around the fluorescent dye. The electric field enhancement caused by the localized surface plasmon resonance of metal nanoparticles 11 under light irradiation increases the excitation probability of the fluorescent dye, resulting in that green fluorescence from the fluorescent dye is enhanced. As a result, the fluorescence intensity to be measured is intensified, and the sensitivity for detecting target DNA 9 can therefore be improved. Accordingly, fluorescence imaging is means particularly suitable for detecting a very small amount of the analyte.

In Example 3, the entire amount of target DNA 9 in well 44 is calculated as around 900 zmol (zeptomoles). It is conceivable that since aggregate 3 has a thickness of around 6 µm, aggregate 3 has a three-dimensional structure in which probe particles 1 and 2 are laid on top of each other. It is conceivable that the amount of target DNA 9 that can be hybridized is around 1/100 of the entire amount. That is, the results of the present Example suggest that target DNA 9 is detectable in a very small amount of around 9 zmol.

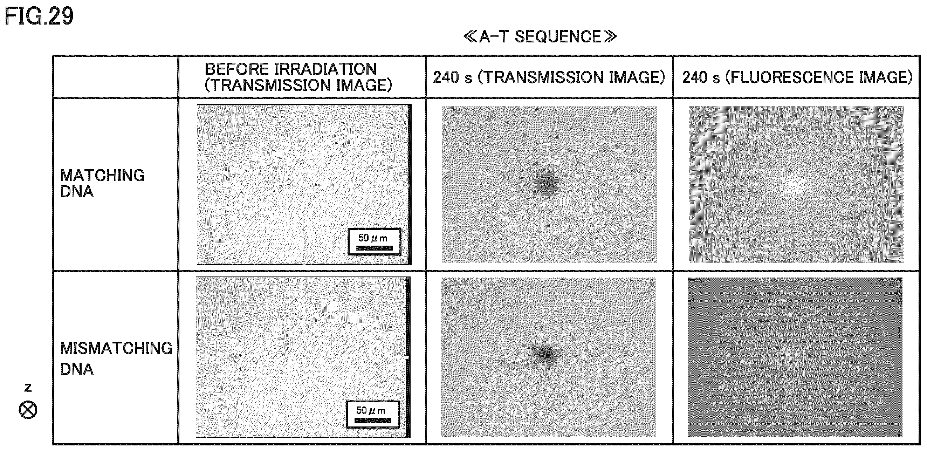

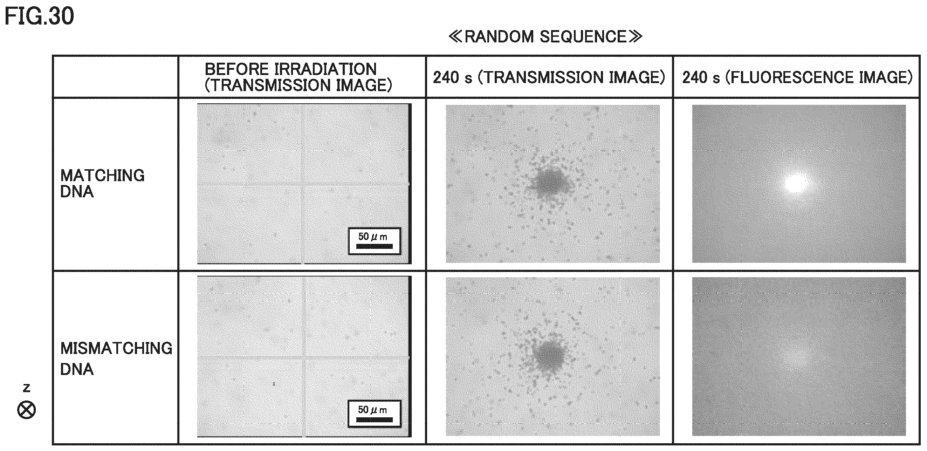

Figs. 29 and 30 are figures showing the dependence of the fluorescence intensity on the base sequence. Fig. 29 shows the observation results in the case where the base sequence of target DNA 9 is an A-T sequence. Fig. 30 shows the observation results in the case where the base sequence of target DNA 9 is a random sequence. Specifically, a matching DNA was 5'-AAA AAA AAA AAA X AAA AAA AAA AAA-3'. A mismatching DNA was Biotin-5'-TTT TTT TTT TTT X TTT TTT TTT TTT-3'. Laser light was irradiated upward using condensing lens 54 provided in an inverted microscope (not shown). The position of the beam waist of the laser light was adjusted so that the beam waist of the laser light was positioned 45 µm over top surface TS of well 44. Condensing lens 54 had a magnification of 40. The intensity of the laser light was 525 mW. The light irradiation time was 240 seconds. The concentration of target DNA 9 was 10 nM. The concentration of probe particles 2 was 5.3 × 10⁸ [particles/mL].

The accumulation area of probe particles 1 and 2 was larger, and the fluorescence intensity was also stronger in the case of using the random sequence (refer to Fig. 30) than in the case of using the A-T sequence (refer to Fig. 29). While the A-T sequence forms only bonding between adenine and thymine, the random sequence can form both bonding between adenine and thymine and bonding between guanine and cytosine. While the bonding between adenine and thymine is double hydrogen bonding, the bonding between guanine and cytosine is triple hydrogen bonding. The bonding strength between guanine and cytosine is therefore stronger than the bonding strength between adenine and thymine. It is conceivable that since the random sequence had a stronger bonding strength, the accumulation area was larger, and the fluorescence intensity was stronger.

### [Example 4]

An attempt to detect target DNA with still higher sensitivity (further improve the detection limit) will be described.

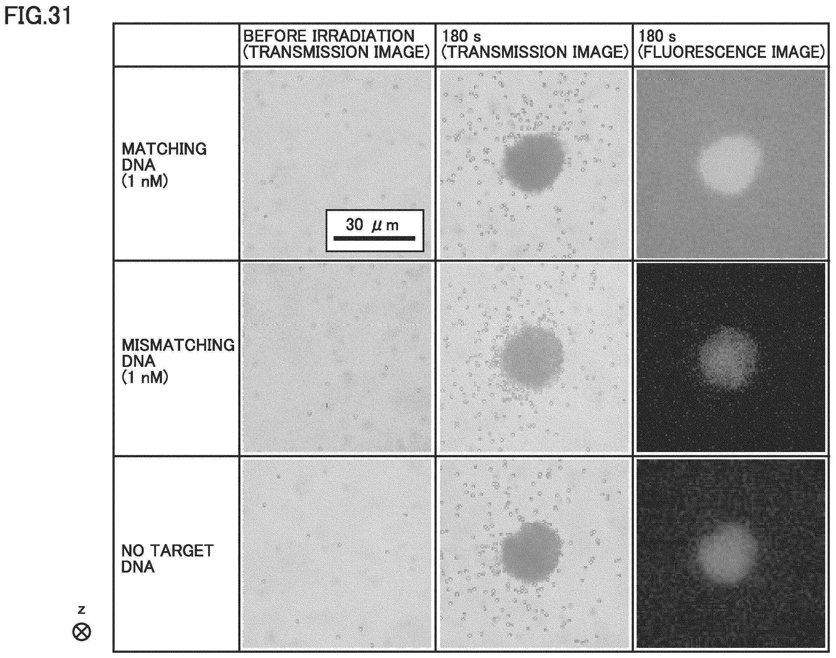

Fig. 31 shows transmission images and fluorescence images of low-concentration samples. The concentration of a matching DNA or a mismatching DNA as target DNA 9 was set in the range of 0 nM to 1000 pM (= 1 nM). The measurement results shown in Fig. 31 are the results of the matching DNA at a concentration of 1000 pM (upper), the results of the mismatching DNA at a concentration of 1000 pM (middle), and the results of no target DNA (lower). The images show transmission images before the laser light irradiation, transmission images after the laser light irradiation, and fluorescence images after the laser light irradiation from left to right sequentially. The light irradiation time was 180 seconds.

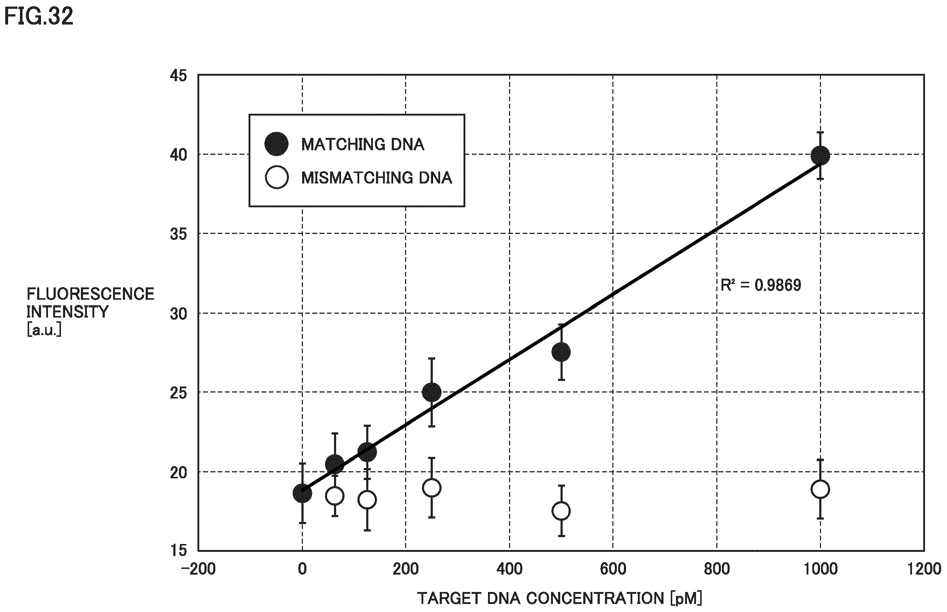

Fig. 32 is a graph summarizing the measurement results of the fluorescence intensities at target DNA 9 concentrations. The abscissae axis indicates the concentration of target DNA 9. The ordinate axis indicates the fluorescence intensity. As shown in Fig. 32, a definite difference in fluorescence intensity between the matching DNA and the mismatching DNA was confirmed at a concentration of 62.5 to 1000 pM (at a concentration by mass of 514.8 [fg/µL] to 7.92 [pg/µL]). Especially with respect to the matching DNA, a linear calibration curve having a coefficient of determination R² of around 1 was obtained. This shows that while Example 3 is targeted at a concentration of 1 nM, 5 nM, or 10 nM (refer to Figs. 18 and 27), Example 4 enables quantitatively evaluating matching DNA at a very low concentration of 1 nM or less.

Let σ the standard deviation, the detection limit was defined as "the minimum amount in which the analyte was not detected in the signal distribution of the background (blank) with a probability of 3σ (≅ 99.7%)" (detection limit = average value of blank ± 3σ), the detection limit of the matching DNA in Example 4 was calculated as 125 to 250 pM ≅ 1.0 to 2.0 [pg/µL]. This detection limit is equivalent to the 100-fold diluted concentration of cell-free DNA (cf DNA), which is used as a biomarker in the field of cancer and reproductive therapy, in a patient-derived sample (blood plasma) (a concentration by mass of 5 to 30 [pg/µL]).

The above-mentioned detection limit is also equivalent to the detection limit of digital PCR (polymerase chain reaction) (a concentration by mass of 166 [fg/µL]). Meanwhile, while digital PCR requires around 5 hours (= 300 minutes), the light irradiation requires 180 seconds (= 3 minutes) in Example 4. The detection time has been shortened by a factor of 100, and that is, the detection has therefore been successfully hastened greatly.

The detection of target DNA with higher sensitivity (and improved speed) can be achieved mainly by three improvements described below.

A first improvement is the change of conditions for irradiating laser light. In Example 3, the beam waist was positioned 45 µm under bottom surface BS of well 44 in the downward irradiation under the defocus conditions (refer to Fig. 8). In Example 4, the beam waist was meanwhile positioned 30 µm over top surface TS of well 44 in the upward irradiation under the defocus conditions. That is, in Example 4, the laser spot size (spot diameter) on top surface TS of well 44 was reduced.

Increasing the laser light irradiation area to a certain degree enables pressing probe particles 2 against top surface TS of well 44 over a large area. Meanwhile, increasing the laser light irradiation area excessively reduces the power density of the laser light to lessen dissipative force acting on probe particles 2. In Example 4, the balance between the laser light irradiation area and the power density was optimized to achieve the condition that strong dissipative force acts on probe particles 2 over a large area. As a result, it is conceivable that the probability of probe particles 2 encountering target DNA 9 further increased.

A second improvement is the adjustment of the correction collar provided in condensing lens 54 that is an objective lens. In Example 3, the correction collar was adjusted to a position of the thickness of cover 42 (corresponding to the thickness of the cover glass, namely around 0.17 mm, in this example). Meanwhile, in Example 4, the correction collar was adjusted to a position calculated by adding the depth of well 44 to the thickness of cover 42 (0.17 mm + 1 mm ≅ 1.2 mm) so that the spherical aberration was corrected according to not only the thickness of cover 42 but also the depth of well 44. It is conceivable that this leads to suitable correction to improve the S/N ratio.

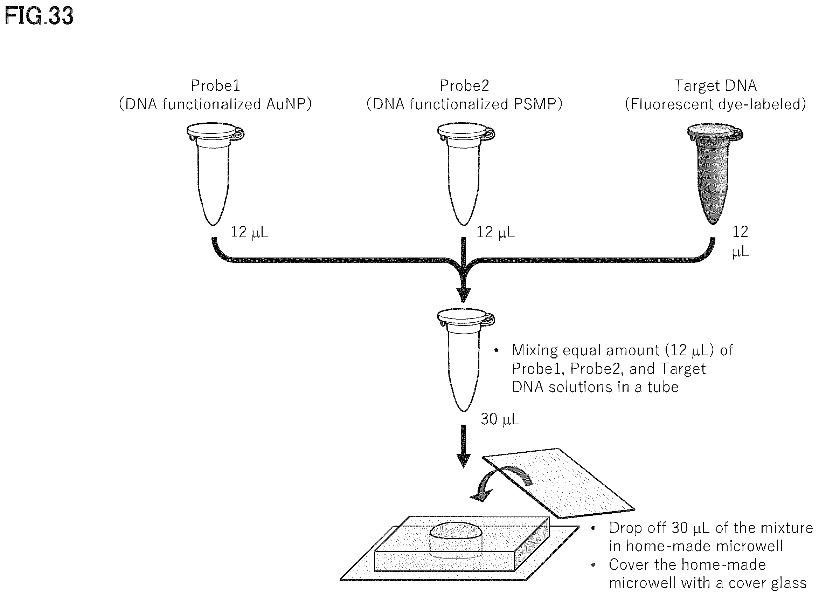

Fig. 33 is an illustration for describing a third improvement. The third improvement is a change in the procedure for preparing the sample. In Example 3, the dispersion of probe particles 1, the dispersion of probe particles 2, and the dispersion of target DNA 9 were separately introduced into well 44. Meanwhile, in Example 4, the above-mentioned 3 dispersions were mixed in a tube, and the mixture was then introduced into well 44. The preliminary mixing enables starting light irradiation under the condition that probe particles 1, probe particles 2, and target DNA 9 are uniformly distributed. This keeps probe particles 2 near probe particles 1 after encountering with target DNA 9 due to Brownian motion, resulting in increasing the probability of probe particles 1 encountering probe particles 2.

### [Example 5]

The results of evaluating detection specificity to the base sequence of target DNA 9 will be described. In this example, 5'-ATG CTC AACTCT-3'-Biotin, containing 12 bases, and SH-5'-TCT CAA CTC GTAT-3', containing 12 bases, were commonly used as the probe DNA. Eight target DNAs 9 containing 24 bases were prepared.

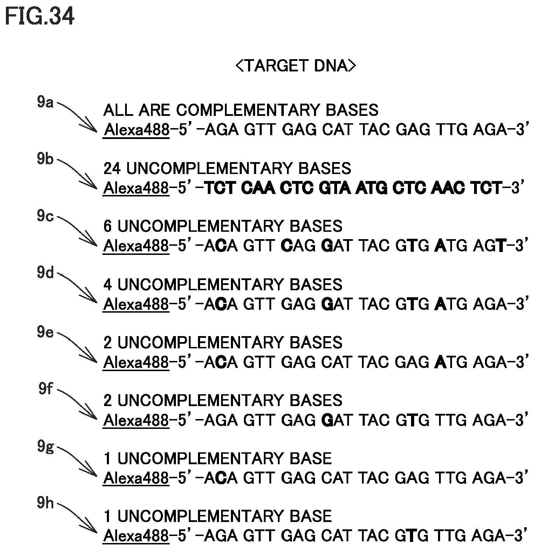

Fig. 34 is an illustration for describing the eight target DNAs 9. Hereinafter, a base complementary to the above-mentioned two probe DNAs is described as a "complementary base". A base that is not complementary to the two probe DNAs is described as a "non-complementary base". In Fig. 34, non-complementary bases are indicated in bold letter.

Target DNA 9a is a matching DNA containing 24 bases that are all complementary. Meanwhile, target DNAs 9b to 9h each contain at least one non-complementary base. In detail, target DNA 9b contains 24 non-complementary bases. Target DNA 9c contains 6 non-complementary bases (and 18 complementary bases). Target DNA 9d contains 4 non-complementary bases (and 20 complementary bases). Target DNAs 9e and 9f each contain 2 non-complementary bases (and 22 complementary bases). Target DNAs 9e and 9f have the 2 non-complementary bases at different positions. Target DNAs 9g and 9h each contain 1 non-complementary base (and 23 complementary bases). Target DNAs 9g and 9h are different in the position of the 1 non-complementary base.

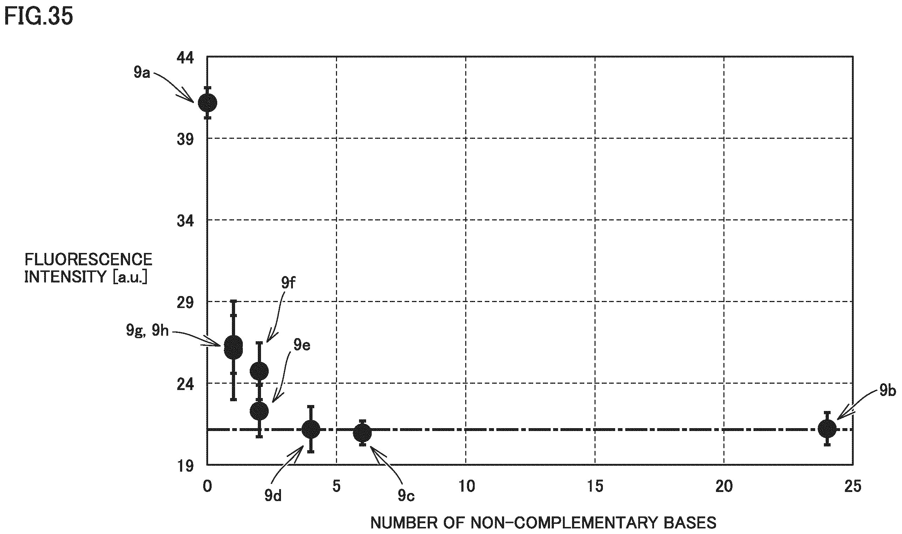

Fig. 35 is a graph summarizing the measurement results of the fluorescence intensities of the eight target DNAs 9. The abscissa axis indicates the number of non-complementary bases. The ordinate axis indicates the fluorescence intensity. All the eight target DNAs 9 were prepared at a concentration of 1 nM. The number of the measurements n was 3. The fluorescence intensity in the case where any target DNA 9 is not contained is indicated with an alternate long and short dash line as the base line, for comparison.

If all the bases were complementary, the fluorescence intensity was remarkably high (refer to 9a). If the number of non-complementary bases was 4 or more, the fluorescence intensity was at the same level as the base line (refer to 9b to 9d). If the number of non-complementary bases was 1 or 2, the fluorescence intensity was high by a narrow margin as compared with the base line (refer to 9e to 9h). Specifically, the difference between the fluorescence intensity in the case where the number of non-complementary bases was 1 and the base line was only around 25% of the difference between the fluorescence intensity in the case where all the bases were complementary bases and the base line. This shows that the method for detecting target DNA 9 according to the present embodiment exhibits high base sequence specificity, and enables detecting especially single nucleotide polymorphisms (SNPs) of target DNA 9.

The photothermal effect of probe particles 1 is considered as one of the reasons why the method for detecting target DNA 9 according to the present embodiment exhibits base sequence specificity. Even though target DNA 9 contains a few non-complementary bases like target DNA 9e to 9h, target DNA 9 can be hybridized to form double strands. As non-complementary bases contained in target DNA 9 increase, the bonding between the probe DNAs and target DNA 9 however becomes weaker, so that the temperature at which double strands dissociate to be single strands (for example, the melting temperature Tm, at which 50% of the double strands dissociate) is low. The photothermal effect of probe particles 1 heats the double strands to high temperature to preferentially dissociate double strands containing many non-complementary bases, so that double strands containing no non-complementary base remain. As a result, target DNA 9a in which all the bases are complementary bases is selectively detected.

### [Example 6]

The detection results of target DNA in a highly viscous sample will be described. A highly viscous sample and a low viscous sample having different liquid crystal concentrations were prepared.

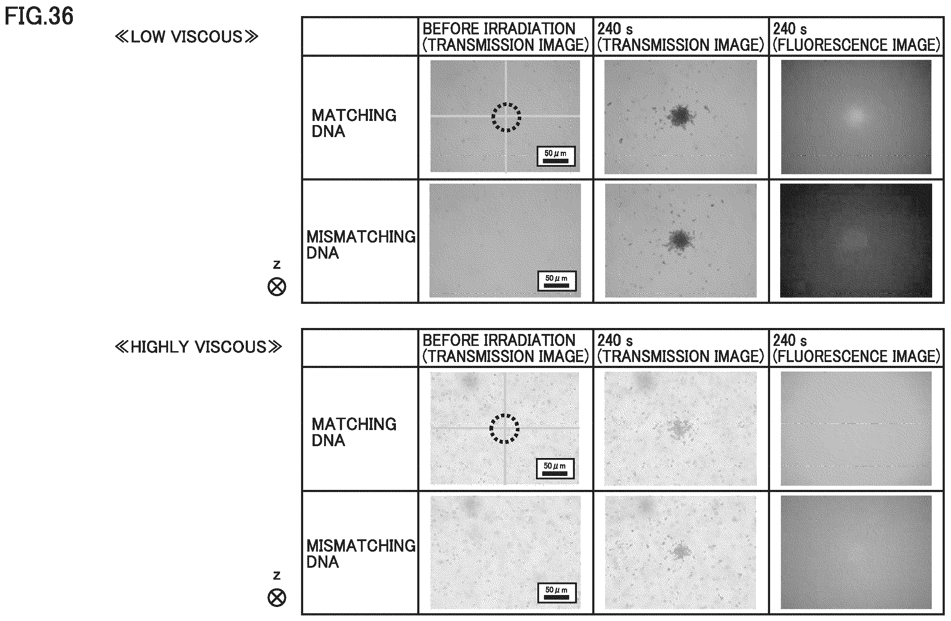

Fig. 36 shows the detection results of target DNA in a highly viscous sample. The highly viscous sample had a liquid crystal concentration of 3.0% by weight, and the low viscous sample had a liquid crystal concentration of 0.3% by weight. The concentration of target DNA 9 (matching DNA) was 1 nM. The concentration of probe particles 2 was 1.58 × 10⁸ [particles/mL]. Laser light was irradiated upward, and the beam waist was positioned 45 µm over top surface TS of well 44. The intensity of the laser light was set at 525 mW, and the irradiation time was set at 240 seconds.

As shown in Fig. 36, fluorescence was also observed in the highly viscous sample. Accordingly, it is found that light-induced acceleration is also achieved in the highly viscous sample. This suggests that target DNA 9 can be detected even in a sample containing impurities.

As mentioned above, in the present embodiment, a hetero-probe method using probe particles 1 containing metal nanoparticles 11 and probe particles 2 in the micrometer order is adopted. This enables detecting target DNA 9 at a very low concentration in the nM (nanomolar) order, in the pM (picomolar) order under the suitably set conditions, within only a short time of several minutes due to the synergistic action of Brownian motion, dissipative force, and thermal convection. That is, the present embodiment enables detecting target DNA 9 with high sensitivity rapidly.

### [Aspects]

Those skilled in the art understand that the above-mentioned illustrative embodiments are specific examples of the following aspects.

### <Item 1>

A method for detecting an analyte, comprising:
Preparing a liquid sample including nanoparticles and microparticles, the nanoparticles each being modified with first host molecules to be specifically bound to the analyte, the microparticles each being modified with second host molecules to be specifically bound to the analyte;
irradiating the liquid sample with non-resonant light outside a wavelength region of electronic resonance of the nanoparticles and outside a wavelength region of electronic resonance of the microparticles; and
detecting the analyte based on an output signal from a light receiver receiving light from the liquid sample,
wherein the preparing includes combining the nanoparticles and the microparticles that are different from each other in size and material so that (i) the nanoparticles each have such a size as to be diffused in the liquid sample by Brownian motion, (ii) the microparticles each have such a size as to receive dissipative force due to Mie scattering of the non-resonant light, and (iii) the nanoparticles each include material that generates thermal convection in the liquid sample by the irradiating the non-resonant light.

### <Item 2>

The method for detecting the analyte according to item 1, wherein the preparing further includes combining the nanoparticles and the microparticles different from each other in concentration so that an average interparticle distance of the nanoparticles is shorter than an average interparticle distance of the microparticles.

### <Item 3>

The method for detecting the analyte according to item 1 or 2, wherein the preparing further includes mixing the nanoparticles and the microparticles uniformly.

### <Item 4>

The method for detecting the analyte according to any one of items 1 to 3, further comprising:
retaining the liquid sample on a substrate; and
adjusting, after the retaining, an irradiation position of the non-resonant light so that a focal point of the non-resonant light is positioned behind the substrate in a non-resonant light propagation direction.

### <Item 5>

The method for detecting the analyte according to any one of items 1 to 3, further comprising:
adjusting an irradiation position of the non-resonant light so that a focal point of the non-resonant light is positioned behind a substrate in a non-resonant light propagation direction; and
retaining, after the adjusting, the liquid sample on the substrate.

### <Item 6>

The method for detecting the analyte according to any one of items 1 to 3, further comprising
retaining the liquid sample in a well provided on a substrate,
wherein the irradiating includes condensing the non-resonant light with a condensing lens including a correction collar, and
the method further comprises adjusting the correction collar so as to correct the spherical aberration in accordance with thickness of the substrate and depth of the well.

### <Item 7>

The method for detecting the analyte according to any one of items 1 to 6,
wherein the analyte is labeled with fluorescent dye,
the detecting includes:
   measuring fluorescence intensity of the liquid sample; and
   detecting the analyte based on a variation in the fluorescence intensity accompanying the irradiating the non-resonant light.

### <Item 8>

The method for detecting the analyte according to item 7,
wherein the analyte is a DNA including multiple bases, and
the detecting includes detecting a single nucleotide polymorphism of the DNA.

### <Item 9>

The method for detecting the analyte according to any one of items 1 to 6,
wherein the detecting includes:
measuring an absorbance spectrum of the liquid sample; and
detecting the analyte based on a variation in the absorbance spectrum accompanying the irradiating the non-resonant light.

### <Item 10>

The method for detecting the analyte according to any one of items 1 to 6,
wherein the detecting includes:
photographing an accumulation region of the accumulated nanoparticles; and microparticles; and
detecting the analyte based on an area of the accumulation region.

### <Item 11>

The method for detecting the analyte according to any one of items 1 to 10,
wherein the liquid sample is a highly viscous liquid sample including impurities,
the detecting includes specifically detecting the analyte from the highly viscous liquid sample.

### <Item 12>

A system for detecting an analyte, the system comprising:
nanoparticles each modified with first host molecules to be specifically bound to the analyte;
microparticles each modified with second host molecules to be specifically bound to the analyte;
a light source that emits non-resonant light outside a wavelength region of electronic resonance of the nanoparticles and outside a wavelength region of electronic resonance of the microparticles;
a light receiver that receives light from a liquid sample irradiated with the non-resonant light and including the nanoparticles and the microparticles; and
a processor that executes detection processing that detects the analyte based on an output signal from the light receiver,
wherein the nanoparticles and the microparticles that are different from each other in size and material are combined so that (i) the nanoparticles each have such a size as to be diffused in the liquid sample by Brownian motion, (ii) the microparticles each have such a size as to receive dissipative force due to Mie scattering of the non-resonant light, and (iii) the nanoparticles each include material that generates thermal convection in the liquid sample by irradiating the non-resonant light.

The scope of the present disclosure is shown not by the description of the above-mentioned embodiments but by CLAIMS.

### REFERENCE SIGNS LIST

1, 2 Probe particle; 11 Metal nanoparticle; 12 Host molecule; 13 Interactive sites; 21 Microparticle; 22 Host molecule; 23 Interactive site; 231 Avidin; 232 Biotin; 3 Aggregate; 4 Detection kit; 41 Substrate; 42 Cover; 43 Spacer; 44 Well; 51 Sample stage; 52 Adjustment mechanism; 53 Laser light source; 54 Condensing lens; 55 Illuminating light source; 55A Excitation light source; 56 Objective lens; 57 Camera; 58 Spectroscope; 59 Optical component; 591 Mirror; 592 Half mirror; 593 Lens; 594 Shutter; 595A, 595B Mirror; 596 Dichroic mirror; 597A, 597B Beam splitter; 598 Lens; 6 Controller; 61 Processor; 62 Memory; 63 Input/output port; 9 Target DNA; 90 Fluorescent dye; 100, 100A Detection system.

## Claims

1. A method for detecting an analyte (9) labeled with fluorescent dye, the method comprising:
preparing a liquid sample (SP) including nanoparticles (11) and microparticles (2), the nanoparticles (11) each being modified with first host molecules (12) to be specifically bound to the analyte (9) , the microparticles (21) each being modified with second host molecules (22) to be specifically bound to the analyte (9),
wherein the nanoparticles (11) and the microparticles (21) that are different from in size and material are combined so that (i) the nanoparticles (11) each have such a size as to be diffused in the liquid sample (SP) by Brownian motion, (ii) the microparticles (21) each have such a size as to receive dissipation force due to Mie scattering of non-resonant light (L1), and (iii) the nanoparticles (11) each include material that generates thermal convection in the liquid sample (SP) due to photothermal effect of irradiating the non-resonant light (L1),
the non-resonant light (L1) is laser light having a wavelength outside a wavelength region of electronic resonance of the nanoparticles (11) and outside a wavelength region of electronic resonance of the microparticles (21), and is equivalent to a diameter of the microparticles (21),
the detection method further comprises:
irradiating the liquid sample (SP) with the non-resonant light (L1) so that the nanoparticles (11) are bound to the microparticles (21) through the first host molecules (12), the analyte (9), and the second host molecules (22) to form an aggregate if the liquid sample (SP) contains the analyte (9) and
detecting the analyte (9) based on an output signal from a light receiver (57) receiving light from the liquid sample (SP), and
the detecting includes
measuring fluorescence intensity of the liquid sample (SP) and
detecting the analyte (9) based on a variation in the fluorescence intensity accompanying the irradiating the non-resonant light (L1).

2. The method for detecting the analyte (9) according to claim 1, wherein the preparing further includes combining the nanoparticles (11) and the microparticles (21) different from each other in concentration so that an average interparticle distance of the nanoparticles (11) is shorter than an average interparticle distance of the microparticles (21).

3. The method for detecting the analyte (9) according to claim 1 or 2, wherein the preparing further includes mixing the nanoparticles (11) and the microparticles (21) uniformly.

4. The method for detecting the analyte (9) according to any one of claims 1 to 3, further comprising:
retaining the liquid sample (SP) on a substrate (4); and
adjusting, after the retaining, an irradiation position of the non-resonant light (L1) so that a focal point of the non-resonant light (L1) is positioned behind the substrate (4) in a non-resonant light propagation direction.

5. The method for detecting the analyte (9) according to any one of claims 1 to 3, further comprising:
adjusting an irradiation position of the non-resonant light (L1) so that a focal point of the non-resonant light (L1) is positioned behind a substrate (4) in the non-resonant light propagation direction; and
retaining, after the adjusting, the liquid sample (SP) on the substrate (4).

6. The method for detecting the analyte (9) according to any one of claims 1 to 3, further comprising:
retaining the liquid sample (SP) in a well (44) provided in a substrate (4),
wherein the irradiating includes condensing the non-resonant light (L1) with a condensing lens (56) including a correction collar, and
the method further comprises: adjusting the correction collar so as to correct the spherical aberration in accordance with thickness of the substrate (4) and depth of the well (44).

7. The method for detecting the analyte (9) according to claim 1,
wherein the analyte (9) is DNA including multiple bases, and
the detecting comprises: detecting a single nucleotide polymorphism of the DNA,
the detecting the single nucleotide polymorphism of the DNA includes heating double strands including a non-complementary base that is not complementary to the multiple bases contained in the DNA to high temperature due to the photothermal effect of the nanoparticles (11) to dissociate the double strands.

8. The method for detecting the analyte (9) according to any one of claims 1 to 3,
wherein the detecting includes
measuring an absorbance spectrum of the liquid sample (SP); and
detecting the analyte (9) based on a variation in the absorbance spectrum accompanying the irradiating the non-resonant light (L1).

9. The method for detecting the analyte (9) according to any one of claims 1 to 3,
wherein the detecting includes
photographing an accumulation region of the accumulated nanoparticles (11) and microparticles (21); and
detecting the analyte (9) based on an area of the accumulation region.

10. The method for detecting the analyte (9) according to any one of claims 1 to 9,
wherein the liquid sample (SP) is a highly viscous liquid sample including impurities,
the detecting includes specifically detecting the analyte (9) from the highly viscous liquid sample.

11. A system (100) for detecting an analyte (9) labeled with fluorescent dye, the system (100) comprising:
nanoparticles (11) each modified with first host molecules (12) to be specifically bound to the analyte (9);
microparticles (21) each modified with second host molecules (22) to be specifically bound to the analyte (9);
a light source (53) that emits non-resonant light (L1) that is laser light having a wavelength that is outside a wavelength region of electronic resonance of the nanoparticles (11) and outside a wavelength region of electronic resonance of the microparticles (21), and is equivalent to a diameter of the microparticles (21);
a light receiver (57) that receives light from a liquid sample (SP) irradiated with the non-resonant light (L1) and including the nanoparticles (11) and the microparticles (21); and
a processor (61) that executes detection processing that detects the analyte (9) based on an output signal from the light receiver (57),
wherein the detection processing is processing that detects, based on a variation in fluorescence intensity of the liquid sample (SP) accompanying the irradiating the non-resonant light (L1), an aggregate formed by binding the nanoparticles (11) to the microparticles (21) through the first host molecules (12), the analyte (9), and the second host molecules (22) if the liquid sample (SP) contains the analyte (9), and
the nanoparticles (11) and the microparticles (21) that are different from each other in size and material are combined so that (i) the nanoparticles (11) each have such a size as to be diffused in the liquid sample (SP) by Brownian motion, (ii) the microparticles (21) each have such a size as to receive dissipation force due to Mie scattering of the non-resonant light (L1), and (iii) the nanoparticles (11) each include material that generates thermal convection in the liquid sample (SP) due to photothermal effect of the irradiating the non-resonant light (L1).

12. The system (100) for detecting the analyte (9) according to claim 11,
wherein the analyte (9) is DNA including multiple bases, and
the detection processing includes processing that heats double strands including a non-complementary base that is not complementary to the multiple bases contained in the DNA to high temperature due to the photothermal effect of the nanoparticles (11) to dissociate the double strands, resulting in detecting a single nucleotide polymorphism of the DNA.

## Patentansprüche

1. Verfahren zum Nachweis eines mit einem Fluoreszenzfarbstoff markierten Analyten (9), wobei das Verfahren umfasst:
Herstellung einer flüssigen Probe (SP), die Nanopartikel (11) und Mikropartikel (21) enthält, wobei die Nanopartikel (11) jeweils mit ersten Wirtsmolekülen (12) modifiziert sind, um spezifisch an den Analyten (9) gebunden zu werden, und die Mikropartikel (21) jeweils mit zweiten Wirtsmolekülen (22) modifiziert sind, um spezifisch an den Analyten (9) gebunden zu werden,
wobei die Nanopartikel (11) und die Mikropartikel (21), die sich in Größe und Material unterscheiden, so kombiniert werden, dass (i) die Nanopartikel (11) jeweils eine solche Größe aufweisen, dass sie durch Brownsche Bewegung in der Flüssigkeitsprobe (SP) diffundieren, (ii) die Mikropartikel (21) jeweils eine solche Größe aufweisen, dass sie eine Dissipationskraft aufgrund der Mie-Streuung von nichtresonantem Licht (L1) aufnehmen, und (iii) die Nanopartikel (11) jeweils Material enthalten, das in der Flüssigkeitsprobe (SP) aufgrund des photothermischen Effekts der Bestrahlung mit dem nichtresonanten Licht (L1) eine thermische Konvektion erzeugt, das nichtresonante Licht (L1) Laserlicht mit einer Wellenlänge außerhalb eines Wellenlängenbereichs der elektronischen Resonanz der Nanopartikel (11) und außerhalb eines Wellenlängenbereichs der elektronischen Resonanz der Mikropartikel (21)ist und einem Durchmesser der Mikropartikel (21) entspricht,
wobei das Verfahren ferner umfasst:
Bestrahlung der flüssigen Probe (SP) mit dem nichtresonanten Licht (L1) so, dass die Nanopartikel (11) über die ersten Wirtsmoleküle (12) an die Mikropartikel (21), den Analyten (9) und die zweiten Wirtsmoleküle (22) gebunden werden, um ein Aggregat zu bilden, wenn die flüssige Probe (SP) den Analyten (9) enthält, und Nachweisen des Analyten (9) auf der Grundlage eines Ausgangssignals eines Lichtempfängers (57), der Licht von der Flüssigkeitsprobe (SP) empfängt, und
wobei das Nachweisen umfasst Messen der Fluoreszenzintensität der Flüssigkeitsprobe (SP) und Nachweisen des Analyten (9) auf der Grundlage einer Änderung der Fluoreszenzintensität, die mit der Bestrahlung durch das nichtresonante Licht (L1) einhergeht.

2. Verfahren zum Nachweis des Analyten (9) gemäß Anspruch 1, wobei das Herstellen ferner das Zusammenbringen der Nanopartikel (11) und der Mikropartikel (21), die sich in ihrer Konzentration voneinander unterscheiden, umfasst, so dass ein durchschnittlicher Partikelabstand der Nanopartikel (11) kürzer ist als ein durchschnittlicher Partikelabstand der Mikropartikel (21).

3. Verfahren zum Nachweis des Analyten (9) gemäß Anspruch 1 oder 2, wobei das Herstellen ferner das gleichmäßige Vermischen der Nanopartikel (11) und der Mikropartikel (21) umfasst.

4. Verfahren zum Nachweis des Analyten (9) gemäß einem der Ansprüche 1 bis 3, das ferner umfasst:
das Halten der flüssigen Probe (SP) auf einem Substrat (4); und
das Einstellen einer Bestrahlungsposition des nichtresonanten Lichts (L1) nach dem Halten, so dass ein Brennpunkt des nichtresonanten Lichts (L1) hinter dem Substrat (4) in einer Ausbreitungsrichtung des nichtresonanten Lichts positioniert ist.

5. Verfahren zum Nachweis des Analyten (9) gemäß einem der Ansprüche 1 bis 3, das ferner umfasst:
das Einstellen einer Bestrahlungsposition des nichtresonanten Lichts (L1), so dass ein Brennpunkt des nichtresonanten Lichts (L1) hinter einem Substrat (4) in der Ausbreitungsrichtung des nichtresonanten Lichts positioniert ist; und
nach der Anpassung das Halten der Flüssigkeitsprobe (SP) auf dem Substrat (4).

6. Verfahren zum Nachweis des Analyten (9) gemäß einem der Ansprüche 1 bis 3, das ferner umfasst:
das Halten der flüssigen Probe (SP) in einer in einem Substrat (4) vorgesehenen Vertiefung (44),
wobei das Bestrahlen das Bündeln des nichtresonanten Lichts (L1) mit einer Bündellinse (56) umfasst, die einen Korrekturkragen aufweist, und
das Verfahren ferner umfasst: Einstellen des Korrekturkragens, um die sphärische Aberration entsprechend der Dicke des Substrats (4) und der Tiefe der Vertiefung (44) zu korrigieren.

7. Verfahren zum Nachweis des Analyten (9) gemäß Anspruch 1, wobei der Analyt (9) DNA ist, die mehrere Basen enthält, und
das Nachweisen umfasst: Nachweisen eines Einzelnukleotid-Polymorphismus der DNA,
wobei das Nachweisen des Einzelnukleotid-Polymorphismus der DNA das Erhitzen von Doppelsträngen, die eine nichtkomplementäre Base enthalten, die nicht komplementär zu den in der DNA enthaltenen mehreren Basen ist, auf eine hohe Temperatur aufgrund des photothermischen Effekts der Nanopartikel (11) umfasst, um die Doppelstränge zu dissoziieren.

8. Verfahren zum Nachweis des Analyten (9) gemäß einem der Ansprüche 1 bis 3, wobei das Nachweisen umfasst:
Messen eines Absorptionsspektrums der flüssigen Probe (SP); und
Nachweisen des Analyten (9) auf der Grundlage einer Veränderung des Absorptionsspektrums, die mit der Bestrahlung durch das nichtresonante Licht (L1) einhergeht.

9. Verfahren zum Nachweis des Analyten (9) gemäß einem der Ansprüche 1 bis 3, wobei das Nachweisen umfasst
Fotografieren eines Ansammlungsbereichs der angesammelten Nanopartikel (11) und Mikropartikel (21); und
Nachweisen des Analyten (9) auf der Grundlage einer Fläche des Ansammlungsbereichs.

10. Verfahren zum Nachweis des Analyten (9) gemäß einem der Ansprüche 1 bis 9,
wobei die Flüssigkeitsprobe (SP) eine hochviskose Flüssigkeitsprobe ist, die Verunreinigungen enthält,
und das Nachweisen den spezifischen Nachweis des Analyten (9) aus der hochviskosen Flüssigkeitsprobe umfasst.

11. System (100) zum Nachweis eines mit einem Fluoreszenzfarbstoff markierten Analyten (9), wobei das System (100) umfasst:
Nanopartikel (11), die jeweils mit ersten Wirtsmolekülen (12) modifiziert sind, um spezifisch an den Analyten (9) gebunden zu werden;
Mikropartikel (21), die jeweils mit zweiten Wirtsmolekülen (22) modifiziert sind, um spezifisch an den Analyten (9) gebunden zu werden;
eine Lichtquelle (53), die nichtresonantes Licht (L1) emittiert, bei dem es sich um Laserlicht mit einer Wellenlänge handelt, die außerhalb eines Wellenlängenbereichs der elektronischen Resonanz der Nanopartikel (11) und außerhalb eines Wellenlängenbereichs der elektronischen Resonanz der Mikropartikel (21) liegt und einem Durchmesser der Mikropartikel (21) entspricht;
einen Lichtempfänger (57), der Licht von einer Flüssigkeitsprobe (SP) empfängt, die mit dem nichtresonanten Licht (L1) bestrahlt wurde und die Nanopartikel (11) sowie die Mikropartikel (21) enthält; und
einen Prozessor (61), der eine Detektionsverarbeitung ausführt, die den Analyten (9) auf der Grundlage eines Ausgangssignals des Lichtempfängers (57) detektiert,
wobei die Detektionsverarbeitung ein Vorgang ist, der auf der Grundlage einer Änderung der Fluoreszenzintensität der Flüssigkeitsprobe (SP), die mit der Bestrahlung mit nichtresonantem Licht (L1) einhergeht, ein Aggregat detektiert, das durch Bindung der Nanopartikel (11) an die Mikropartikel (21) über die ersten Wirtsmoleküle (12), den Analyten (9) und die zweiten Wirtsmoleküle (22) gebildet wird, sofern die Flüssigkeitsprobe (SP) den Analyten (9) enthält, und
die Nanopartikel (11) und die Mikropartikel (21), die sich in Größe und Material voneinander unterscheiden, so kombiniert sind, dass (i) die Nanopartikel (11) jeweils eine solche Größe aufweisen, dass sie durch Brownsche Bewegung in der Flüssigkeitsprobe (SP) diffundieren, (ii) die Mikropartikel (21) jeweils eine solche Größe aufweisen, dass sie eine Dissipationskraft aufgrund der Mie-Streuung des nichtresonanten Lichts (L1) erfahren, und (iii) die Nanopartikel (11) jeweils Material enthalten, das aufgrund des photothermischen Effekts der Bestrahlung mit dem nichtresonanten Licht (L1) eine thermische Konvektion in der Flüssigkeitsprobe (SP) erzeugt.

12. System (100) zum Nachweis des Analyten (9) gemäß Anspruch 11, wobei der Analyt (9) DNA ist, die mehrere Basen enthält, und
die Detektionsverarbeitung einen Schritt umfasst, bei dem Doppelstränge, die eine nichtkomplementäre Base enthalten, die zu den in der DNA enthaltenen mehreren Basen nicht komplementär ist, aufgrund des photothermischen Effekts der Nanopartikel (11) auf eine hohe Temperatur erhitzt werden, um die Doppelstränge zu dissoziieren, was zum Nachweis eines Einzelnukleotid-Polymorphismus der DNA führt.

## Revendications

1. Procédé de détection d'un analyte (9) marqué par un colorant fluorescent, ledit procédé comprenant :
la préparation d'un échantillon liquide (SP) comprenant des nanoparticules (11) et des microparticules (21), les nanoparticules (11) étant chacune modifiées par des premières molécules hôtes (12) afin de se lier spécifiquement à l'analyte (9), les microparticules (21) étant chacune modifiées par des secondes molécules hôtes (22) afin de se lier spécifiquement à l'analyte (9),
dans lequel les nanoparticules (11) et les microparticules (21), qui diffèrent par leur taille et leur matériau, sont combinées de telle sorte que (i) les nanoparticules (11) aient chacune une taille telle qu'elles puissent se diffuser dans l'échantillon liquide (SP) par mouvement brownien, (ii) les microparticules (21) aient chacune une taille telle qu'elles subissent une force de dissipation due à la diffusion de Mie de la lumière non résonante (L1), et (iii) les nanoparticules (11) comprennent chacune un matériau qui génère une convection thermique dans l'échantillon liquide (SP) en raison de l'effet photothermique résultant de l'irradiation par la lumière non résonante (L1),
la lumière non résonante (L1) est une lumière laser ayant une longueur d'onde située en dehors d'une région de longueurs d'onde de résonance électronique des nanoparticules (11) et en dehors d'une région de longueurs d'onde de résonance électronique des microparticules (21), et est équivalente au diamètre des microparticules (21),
le procédé de détection comprend en outre :
l'irradiation de l'échantillon liquide (SP) avec la lumière non résonante (L1) de telle sorte que les nanoparticules (11) soient liées aux microparticules (21) par l'intermédiaire des premières molécules hôtes (12), à l'analyte (9) et à des secondes molécules hôtes (22) pour former un agrégat si l'échantillon liquide (SP) contient l'analyte (9) et
la détection de l'analyte (9) sur la base d'un signal de sortie provenant d'un récepteur de lumière (57) recevant la lumière provenant de l'échantillon liquide (SP), et
la détection comprenant
la mesure de l'intensité de fluorescence de l'échantillon liquide (SP) et
la détection de l'analyte (9) sur la base d'une variation de l'intensité de fluorescence accompagnant l'irradiation par la lumière non résonante (L1).

2. Procédé de détection de l'analyte (9) selon la revendication 1, dans lequel l'étape de préparation comprend en outre la combinaison des nanoparticules (11) et des microparticules (21), dont les concentrations sont différentes, de telle sorte que la distance interparticulaire moyenne des nanoparticules (11) soit inférieure à la distance interparticulaire moyenne des microparticules (21).

3. Procédé de détection de l'analyte (9) selon la revendication 1 ou 2, dans lequel
l'étape de préparation comprend en outre le mélange uniforme des nanoparticules (11) et des microparticules (21).

4. Procédé de détection de l'analyte (9) selon l'une quelconque des revendications 1 à 3, comprenant en outre :
la rétention de l'échantillon liquide (SP) sur un substrat (4) ; et
le réglage, après la rétention, d'une position d'irradiation de la lumière non résonante (L1) de telle sorte qu'un point focal de la lumière non résonante (L1) soit positionné derrière le substrat (4) dans une direction de propagation de la lumière non résonante.

5. Procédé de détection de l'analyte (9) selon l'une quelconque des revendications 1 à 3, comprenant en outre :
le réglage d'une position d'irradiation de la lumière non résonante (L1) de telle sorte qu'un point focal de la lumière non résonante (L1) soit positionné derrière un substrat (4) dans la direction de propagation de la lumière non résonante ; et
le maintien, après l'ajustement, de l'échantillon liquide (SP) sur le substrat (4).

6. Procédé de détection de l'analyte (9) selon l'une quelconque des revendications 1 à 3, comprenant en outre :
le maintien de l'échantillon liquide (SP) dans un puits (44) prévu dans un substrat (4), dans lequel l'irradiation comprend la condensation de la lumière non résonante (L1) à l'aide d'une lentille de condensation (56) comprenant une bague de correction, et le procédé comprend en outre : le réglage de la bague de correction de manière à corriger l'aberration sphérique en fonction de l'épaisseur du substrat (4) et de la profondeur du puits (44).

7. Procédé de détection de l'analyte (9) selon la revendication 1, dans lequel l'analyte (9) est un ADN comprenant plusieurs bases, et
la détection comprend : la détection d'un polymorphisme mononucléotidique de l'ADN,
la détection du polymorphisme mononucléotidique de l'ADN comprenant le chauffage des doubles brins comprenant une base non complémentaire qui n'est pas complémentaire des multiples bases contenues dans l'ADN à une température élevée grâce à l'effet photothermique des nanoparticules (11) afin de dissocier les doubles brins.

8. Procédé de détection de l'analyte (9) selon l'une quelconque des revendications 1 à 3, dans lequel la détection comprend
la mesure d'un spectre d'absorbance de l'échantillon liquide (SP) ; et
la détection de l'analyte (9) sur la base d'une variation du spectre d'absorbance accompagnant l'irradiation par la lumière non résonante (L1).

9. Procédé de détection de l'analyte (9) selon l'une quelconque des revendications 1 à 3, dans lequel la détection comprend
la prise d'une photographie d'une zone d'accumulation des nanoparticules (11) et des microparticules (21) accumulées ; et
la détection de l'analyte (9) sur la base d'une surface de la zone d'accumulation.

10. Procédé de détection de l'analyte (9) selon l'une quelconque des revendications 1 à 9, dans lequel l'échantillon liquide (SP) est un échantillon liquide à haute viscosité contenant des impuretés,
la détection comprenant la détection spécifique de l'analyte (9) à partir de l'échantillon liquide à haute viscosité.

11. Système (100) destiné à détecter un analyte (9) marqué avec un colorant fluorescent, le système (100) comprenant :
des nanoparticules (11) modifiées chacune par des premières molécules hôtes (12) afin d'être spécifiquement liées à l'analyte (9) ;
des microparticules (21) modifiées chacune par des secondes molécules hôtes (22) afin de se lier spécifiquement à l'analyte (9) ;
une source lumineuse (53) qui émet une lumière non résonante (L1), à savoir une lumière laser ayant une longueur d'onde qui se situe en dehors d'une région de longueurs d'onde de résonance électronique des nanoparticules (11) et en dehors d'une région de longueurs d'onde de résonance électronique des microparticules (21), et qui est équivalente au diamètre des microparticules (21) ;
un récepteur de lumière (57) qui reçoit la lumière provenant d'un échantillon liquide (SP) irradié par la lumière non résonante (L1) et comprenant les nanoparticules (11) et les microparticules (21) ; et
un processeur (61) qui exécute un traitement de détection permettant de détecter l'analyte (9) sur la base d'un signal de sortie provenant du récepteur de lumière (57), dans lequel le traitement de détection est un traitement qui détecte, sur la base d'une variation de l'intensité de fluorescence de l'échantillon liquide (SP) accompagnant l'irradiation par la lumière non résonante (L1), un agrégat formé par la liaison des nanoparticules (11) aux microparticules (21) par l'intermédiaire des premières molécules hôtes (12), l'analyte (9), et les secondes molécules hôtes (22) si l'échantillon liquide (SP) contient l'analyte (9), et
les nanoparticules (11) et les microparticules (21), qui diffèrent les unes des autres par leur taille et leur matériau, sont combinées de telle sorte que (i) les nanoparticules (11) aient chacune une taille telle qu'elles puissent se diffuser dans l'échantillon liquide (SP) par mouvement brownien, (ii) les microparticules (21) aient chacune une taille telle qu'elles subissent une force de dissipation due à la diffusion de Mie de la lumière non résonante (L1), et (iii) les nanoparticules (11) comprennent chacune un matériau qui génère une convection thermique dans l'échantillon liquide (SP) en raison de l'effet photothermique de l'irradiation par la lumière non résonante (L1).

12. Système (100) destiné à détecter l'analyte (9) selon la revendication 11, dans lequel l'analyte (9) est un ADN comprenant plusieurs bases, et
le traitement de détection comprend un traitement qui chauffe à haute température les doubles brins comprenant une base non complémentaire qui n'est pas complémentaire des plusieurs bases contenues dans l'ADN, en raison de l'effet photothermique des nanoparticules (11), afin de dissocier les doubles brins, ce qui permet de détecter un polymorphisme mononucléotidique de l'ADN.
